# EUROPÄISCHE PATENTANMELDUNG

(11) **EP 1 972 323 A2**
(43) Veröffentlichungstag der Anmeldung: **24.09.2008**
(21) Anmeldenummer: 07023637.7
(22) Anmeldetag: 06.12.2007
(51) Int. Cl.: A61K 8/06, A61K 8/49, A61K 8/35, A61K 8/46, A61Q 5/10, A61Q 17/04

(54) **Haarfärbemittel**

(30) Priorität: 23.03.2007 DE 102007014630
(71) Anmelder: Henkel AG & Co. KGaA, 40589 Düsseldorf (DE)
(72) Erfinder: Erkens, Udo, 41468 Neuss (DE); Reichert, Anja, 40629 Düsseldorf (DE)

(57) **Zusammenfassung**

Färbemittel, die eine wässrige Phase, mindestens einen Fettstoff als Fettphase, mindestens einen organischen UV-Absorber und mindestens eine farbverändernde Komponente enthalten, wobei der UV-Absorber in die Fettphase eingearbeitet ist und die farbverändernden Komponenten sich in der wässrigen Phase befinden, bieten einen Schutz vor dem Ausbleichen der Färbung durch UV-Strahlung.

## Beschreibung

Die vorliegende Erfindung betrifft Färbemittel, die eine wässrige Phase, mindestens einen Fettstoff als Fettphase, mindestens einen organischen UV-Absorber und mindestens eine farbverändernde Komponente enthalten, wobei mindestens ein UV-Absorber in die Fettphase eingearbeitet ist und die farbverändernden Komponenten sich in der wässrigen Phase befinden. Ein Verfahren zur Herstellung dieser Mittel, sowie die Verwendung dieser Mittel zur Farbveränderung keratinhaltiger Fasern, insbesondere menschlichem Haar und gleichzeitig zum Schutz der Färbung vor dem Ausbleichen durch Licht, sind ebenso Gegenstand der Erfindung.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im Wesentlichen vier Typen von Haarfärbemitteln von Bedeutung:
Für permanente, intensive Färbungen mit entsprechenden Echtheitseigenschaften werden sogenannte Oxidationsfärbemittel verwendet. Solche Haarfärbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N,N'-bis(4-aminophenyl)-diamino-propan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5- Dihydroxynaphthalin, 2,7- Dihydroxynaphthalin und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methyl resorcin , 5-Methylresorcin und 2-Methyl-4-chlor-5-aminophenol.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Schließlich hat ein weiteres Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Eine weitere Möglichkeit keratinhaltige Fasern zu färben, bietet die Verwendung von Färbemitteln, die eine Kombination aus Komponente
- A: Verbindungen, die eine reaktive Carbonylgruppe enthalten mit Komponente
- B: Verbindungen, ausgewählt aus (a) CH-aciden Verbindungen und (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen
enthalten. Die resultierenden Färbungen besitzen teilweise Farbechtheiten auf der keratinhaltigen Faser, die mit denen der Oxidationsfärbung vergleichbar sind. Das mit der schonenden Oxofärbung erzielbare Nuancenspektrum ist sehr breit und die erhaltene Färbung weist oftmals eine akzeptable Brillanz und Farbtiefe auf. Die vorgenannten Komponenten A und B, im weiteren als Oxofarbstoffvorprodukte bezeichnet, sind im allgemeinen selbst keine Farbstoffe, und eignen sich daher jede für sich genommen allein nicht zur Färbung keratinhaltiger Fasern. In Kombination bilden sie in einem nichtoxidativen Prozess Farbstoffe aus. Unter Verbindungen der Komponente B können allerdings auch entsprechende Oxidationsfarbstoffvorprodukte vom Entwickler- und/oder Kupplertyp mit oder ohne Einsatz eines Oxidationsmittels Verwendung finden. Somit läßt sich die Methode der Oxofärbung ohne weiteres mit dem oxidativen Färbesystem kombinieren.

Der Verbraucher wünscht sich, dass die Färbung keratinhaltiger Fasern, insbesondere menschlicher Haare, gegen diverse Umwelteinflüsse stabil bleibt. Die Färbungen sollen insbesondere gegen Licht, Reibung, Schweiß, Wäschen und chemische Behandlung (wie beispielsweise eine in der Haarbehandlung übliche Anwendung von Dauerwellmitteln) ihre Farbbrillanz und Nuance beibehalten. Die Anwendung der Färbemittel soll demnach eine intensive, brillante und farbstabile Färbung ergeben, die gleichmäßig auf die keratinhaltigen Fasern aufzieht.

In der Druckschrift WO-A1-98/041186 werden oxidative Haarfärbemittel beschrieben, die Oxidationsfarbstoffvorprodukte, ein 2-Hydroxyphenylbenzotriazol-Derivat als UV-Absorber, ein Tensid und Wasser enthalten.

Das Patent EP-B1-437 006 betrifft ein Verfahren zur Behandlung von gefärbtem Haar, in dem das Haar mit einem Shampoo, enthaltend 0,1 bis 5 Gew.-% 2,2',4,4'-Tetrahydroxybenzophenon, Tensid, Haarsubstantivträger und Wasser behandelt wird.

Es wurde überraschender Weise gefunden, dass sich die Färbung keratinhaltiger Fasern gegen die Einwirkung von UV-Licht in verbessertem Maße stabilisieren läßt, wenn das wasserhaltige Färbemittel neben den farbverändernden Komponenten mindestens einen UV-Absorber enthält, der in eine Fettphase eingearbeitet ist und sich die farbverändernden Komponenten in der wässrigen Phase befinden. Die Färbekraft des Färbemittels wird durch die Gegenwart des UV-Filters nicht beeinträchtigt.

Gegenstand der Erfindung ist daher ein Mittel zur Färbung keratinhaltiger Fasern, insbesondere menschlicher Haare, enthaltend
(i) eine wässrige Phase,
(ii) mindestens einen Fettstoff als Fettphase,
(iii) mindestens einen organischen UV-Absorber und
(iv) mindestens eine farbverändernde Komponente,
mit der Maßgabe, dass sich mindestens ein organischer UV-Absorber in mindestens einem Fettstoff der Fettphase befindet und die farbverändernden Komponenten sich in der wässrigen Phase befinden.

Unter keratinhaltigen Fasern sind Wolle, Pelze, Federn und insbesondere menschliche Haare zu verstehen. Die erfindungsgemäße Kombination kann prinzipiell aber auch auf anderen Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

Die erfindungsgemäßen Mittel liegen bevorzugt als Dispersion des Fettstoffes und/oder als Wasser-in-ÖI-Emulsion beziehungsweise als ÖI-in-Wasser-Emulsion vor, wobei hier die Ölphase der Emulsionen unter anderem die Fettstoffe der Fettphase ausmachen.

Unter einer wässrigen Phase ist im Sinne der Erfindung eine wasserhaltige, bei Standardbedingungen (101325 Pa und 25°C) flüssige Phase zu verstehen. Diese Phase enthält bevorzugt mindestens 30 Gew.-% Wasser, besonders bevorzugt mindestens 50 Gew.% Wasser, jeweils bezogen auf das Gewicht des gesamten Färbemittels.

Die wässrige Phase kann zusätzlich mindestens ein in der wässrigen Phase bei Standardbedingungen lösliches organisches Lösemittel enthalten. Diese organischen Lösemittel können bevorzugt zu 1 bis 70 Gew.-%, besonders bevorzugt zu 1 bis 25 Gew.-%, jeweils bezogen auf das Gewicht des gesamten Mittels, in der erfindungsgemäßen wässrigen Phase enthalten sein. Entsprechend einsetzbare organische Lösemittel sind bevorzugt C₁-C₄-Alkohole (insbesondere Ethanol und/oder Isopropanol), Methoxybutanol, Benzylalkohol, Ethyldiglykol oder (C₂ bis C₆)-Alkandiole (wie bevorzugt 1,2-Propylenglykol).

Unter dem Merkmal, dass sich die farbverändernden Komponenten in der wässrigen Phase befinden, ist erfindungsgemäß zu verstehen, dass mehr als 50 Gew.-% (besonders bevorzugt mehr als 75 Gew.-%, ganz besonders bevorzugt mehr als 90 Gew.-%) aller im Färbemittel enthaltenen farbverändernden Komponenten sich in der wässrigen Phase befinden.

Unter den erfindungsgemäßen Fettstoffen sind Verbindungen zu verstehen, die von den farbverändernden Komponenten und den UV-Absorbern verschieden sind und sich unter Standardbedingungen zu weniger als 1 g in 1 Liter Wasser lösen. Erfindungsgemäß bevorzugte Fettstoffe weisen einen Schmelzpunkt von weniger als 150°C, insbesondere von weniger als 100°C, ganz bevorzugt von weniger als 65 °C, auf. Dabei ist es wiederum erfindungsgemäß bevorzugt, wenn die Fettstoffe, in die der organische UV-Absorber eingearbeitet ist, bei Standardbedingungen (101325 Pa und 25°C) als Feststoff vorliegt.

Besonders bevorzugte Fettstoffe werden ausgewählt aus mindestens einer Verbindung aus der Gruppe, die gebildet wird, aus Silikonen, Fettsäuren, Fettalkoholen, natürlichen oder synthetischen Wachsen und natürlichen oder synthetischen kosmetischen Ölkomponenten, sowie Mischungen von Verbindungen aus diesen Stoffklassen. Die Fettstoffe können sowohl in fester Form als auch flüssig in der wässrigen Phase des erfindungsgemäßen Mittels dispergiert vorliegen. Erfindungsgemäß bevorzugte Fettstoffe aus obiger Liste weisen einen Schmelzpunkt von weniger als 150°C, insbesondere von weniger als 100°C, auf. Diese bevorzugte Auswahl gilt auch für die nachfolgend genannten Fettstoffe *(vide infra).*

Es ist wiederum bevorzugt, die Fettstoffe aus den Fettalkoholen und/oder den natürlichen oder synthetischen Wachsen und/oder den natürlichen oder synthetischen kosmetischen Ölkomponenten auszuwählen.

Die Fettstoffe sind vorzugsweise in Mengen von 0,05 bis 45 Gew.-%, bevorzugt von 0,2 bis 35 Gew.-%, besonders bevorzugt von 2,0 Gew.-% bis 25 Gew.-%, jeweils bezogen auf das Gewicht des Färbemittels, enthalten. Spezifische Fettstoffe weisen eine wiederum weiterhin ganz besonders bevorzugte Einsatzmenge auf *(vide infra).*

Insbesondere bevorzugt werden die Silikone ausgewählt, aus mindestens einem Vertreter aus der Liste, die gebildet wird aus:
a) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
b) Polysiloxanen, die in ihrer allgemeinen Struktur eine oder mehrere organofunktionelle Gruppen enthalten, die ausgewählt sind unter:
   1. substituierten oder unsubstituierten aminierten Gruppen;
   2. (per)fluorierten Gruppen;
   3. Thiolgruppen;
   4. Carboxylatgruppen;
   5. hydroxylierten Gruppen;
   6. alkoxylierten Gruppen;
   7. Acyloxyalkylgruppen;
   8. amphoteren Gruppen;
   9. Bisulfitgruppen;
   10. Hydroxyacylaminogruppen;
   11. Carboxygruppen;
   12. Sulfonsäuregruppen; und
   13. Sulfat- oder Thiosulfatgruppen;
c) linearen Polysiloxan(A)- Polyoxyalkylen(B)- Blockcopoylmeren vom Typ (A-B)ₙ mit n > 3;
d) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
e) gepfropften Silikon polymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
f) oder deren Gemischen.

Besonders bevorzugte erfindungsgemäße kosmetische oder dermatologische Zubereitungen sind dadurch gekennzeichnet, dass sie mindestens ein Silikon der Formel (Si-1)

(CH₃)₃Si-[O-Si(CH₃)₂]ₓ O-Si(CH₃)₃ (Si-1),

enthalten, in der x für eine Zahl von 0 bis 100, vorzugsweise von 0 bis 50, weiter bevorzugt von 0 bis 20 und insbesondere 0 bis 10, steht.

Die erfindungsgemäß bevorzugten kosmetischen oder dermatologischen Zubereitungen enthalten ein Silikon der vorstehenden Formel (Si-1). Diese Silikone werden nach der INCI-Nomenklatur als Dimethicone bezeichnet. Es werden im Rahmen der vorliegenden Erfindung als Silikon der Formel (Si-1) vorzugsweise die Verbindungen:
(CH₃)₃Si-O-Si(CH₃)₃
(CH₃)₃Si-O-(CH₃)₂Si-O-Si(CH₃)₃
(CH₃)₃Si-[O-(CH₃)₂Si]₂-O-Si(CH₃)₃
(CH₃)₃Si-[O-(CH₃)₂Si]₃-O-Si(CH₃)₃
(CH₃)₃Si-[O-(CH₃)₂Si]₄-O-Si(CH₃)₃
(CH₃)₃Si-[O-(CH₃)₂Si]₅-O-Si(CH₃)₃
(CH₃)₃Si-[O-(CH₃)₂Si]₆-O-Si(CH₃)₃
(CH₃)₃Si-[O-(CH₃)₂Si]₇-O-Si(CH₃)₃
(CH₃)₃Si-[O-(CH₃)₂Si]₈-O-Si(CH₃)₃
(CH₃)₃Si-[O-(CH₃)₂Si]₉-O-Si(CH₃)₃
(CH₃)₃Si-[O-(CH₃)₂Si]₁₀-O-Si(CH₃)₃
(CH₃)₃Si-[O-(CH₃)₂Si]₁₁-O-Si(CH₃)₃
(CH₃)₃Si-[O-(CH₃)₂Si]₁₂-O-Si(CH₃)₃
(CH₃)₃Si-[O-(CH₃)₂Si]₁₃-O-Si(CH₃)₃
(CH₃)₃Si-[O-(CH₃)₂Si]₁₄-O-Si(CH₃)₃
(CH₃)₃Si-[O-(CH₃)₂Si]₁₅-O-Si(CH₃)₃
(CH₃)₃Si-[O-(CH₃)₂Si]₁₆-O-Si(CH₃)₃
(CH₃)₃Si-[O-(CH₃)₂Si]₁₇-O-Si(CH₃)₃
(CH₃)₃Si-[O-(CH₃)₂Si]₁₈-O-Si(CH₃)₃
(CH₃)₃Si-[O-(CH₃)₂Si]₁₉-O-Si(CH₃)₃
(CH₃)₃Si-[O-(CH₃)₂Si]₂₀-O-Si(CH₃)₃
eingesetzt, wobei (CH₃)₃Si-O-Si(CH₃)₃, (CH₃)₃Si-O-(CH₃)₂Si-O-Si(CH₃)₃ und/oder (CH₃)₃Si-[O-(CH₃)₂Si]₂-O-Si(CH₃)₃ besonders bevorzugt sind.

Selbstverständlich können auch Mischungen der o.g. Silikone in den bevorzugten erfindungsgemäßen Mitteln enthalten sein.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind.

Besonders bevorzugte erfindungsgemäße Mittel enthalten ein oder mehrere aminofunktionelle Silikone. Solche Silikone können z.B. durch die Formel (Si-2)

M(RₐQ_{b}SiO_{(4-a-b)/2)x}(R_{c}SiO_{(4-c)/2)y}M (Si-2)

Beschrieben werden, wobei in der obigen Formel
- R: ein Kohlenwasserstoff oder ein Kohlenwasserstoffrest mit 1 bis etwa 6 Kohlenstoffatomen ist,
- Q: ein polarer Rest der allgemeinen Formel -R¹HZ ist,
worin
- R¹: eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
- Z: ein organischer, aminofunktioneller Rest ist, der mindestens eine aminofunktionelle Gruppe enthält;
- a: Werte im Bereich von etwa 0 bis etwa 2 annimmt,
- b: Werte im Bereich von etwa 1 bis etwa 3 annimmt,
- a + b: kleiner als oder gleich 3 ist, und
- c: eine Zahl im Bereich von etwa 1 bis etwa 3 ist, und
- x: eine Zahl im Bereich von 1 bis etwa 2.000, vorzugsweise von etwa 3 bis etwa 50 und am bevorzugtesten von etwa 3 bis etwa 25 ist, und
- y: eine Zahl im Bereich von etwa 20 bis etwa 10.000, vorzugsweise von etwa 125 bis etwa 10.000 und am bevorzugtesten von etwa 150 bis etwa 1.000 ist, und
- M: eine geeignete Silikon-Endgruppe ist, wie sie im Stande der Technik bekannt ist, vorzugsweise Trimethylsiloxy.

Nicht einschränkende Beispiele der in Formel (Si-2) durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH ₂-, -CH₂CH₂OCH₂-, -OCH₂CH₂-, - OCH₂ CH₂CH₂-, -CH₂CH(CH₃)C(O)OCHr, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein.

Z ist gemäß Formel (Si-2) ein organischer, aminofunktioneller Rest, enthaltend mindestens eine funktionelle Aminogruppe. Eine mögliche Formel für besagtes Z ist NH(CH₂)_{z}NH₂, worin z eine ganze Zahl von größer gleich 1 ist. Eine andere mögliche Formel für besagtes Z ist -NH(CH₂)_{z}(CH ₂)_{zz}NH, worin sowohl z als auch zz unabhängig voneinander eine ganze Zahl von größer gleich 1 sind, wobei diese Struktur Diamino-Ringstrukturen umfasst, wie Piperazinyl. Besagtes Z ist am bevorzugtesten ein -NHCH₂CH ₂NH₂-Rest. Eine andere mögliche Formel für besagtes Z ist -N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q gemäß Formel (Si-2) ist am bevorzugtesten ein polarer aminofunktioneller Rest der Formel - CH₂CH₂CH₂NHCH₂CH₂NH₂.

In der Formel (Si-2) nimmt a Werte im Bereich von 0 bis 2 an, b nimmt Werte im Bereich von 2 bis 3 an, a + b ist kleiner als oder gleich 3, und c ist eine Zahl im Bereich von 1 bis 3. Das molare Verhältnis der RₐQ_{b} SiO_{(4-a-b)/2}-Einheiten zu den R_{c}SiO _{(4-c)/2}-Einheiten in Formel (Si-2) liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silikone der obigen Formel (Si-2) eingesetzt, dann können die verschiedenen variablen Substituenten in der obigen Formel bei den verschiedenen Silikonkomponenten, die in der Silikonmischung vorhanden sind, verschieden sein.

Bevorzugte erfindungsgemäße kosmetische oder dermatologische Zubereitungen enthalten ein aminofunktionelles Silikon der Formel (Si-3)

R'ₐG₃₋ₐSi(OSiG₂)ₙ-(OSiG_{b}R'_{2-b})ₘ-O-SiG₃₋ₐ-R'a (Si-3),

worin bedeutet:
- G: ist -H, eine Phenylgruppe, -OH, -O-CH₃, -CH₃, -O-CH₂CH₃, -CH₂CH₃, -O-CH₂CH₂CH₃, -CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃;
- a: steht für eine Zahl zwischen 0 und 3, insbesondere 0;
- b: steht für eine Zahl zwischen 0 und 1, insbesondere 1,
- m und n: sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R': ist ein monovalenter Rest ausgewählt aus
-Q-N(R")-CH₂-CH₂-N(R")₂
-Q-N(R")₂
-Q-N⁺(R")₃A⁻
-Q-N⁺H(R")₂ A⁻
-Q-N⁺H₂(R")A⁻
-Q-N(R")-CH₂-CH₂-N⁺R"H₂A⁻,
   wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
   R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Erfindungsgemäß geeignet sind kationische Silikonöle wie beispielsweise die im Handel erhältliche Dow Corning 929 Emulsion (enthaltend ein hydroxylamino-modifiziertes Silikon, das als Amodimethicone bezeichnet wird), DC 2-2078 (Hersteller Dow Corning, INCI-Bezeichung: Aminopropyl Phenyl Trimethicone), DC 5-7113 (Hersteller Dow Corning, INCl-Bezeichnung: Silicone Quaternium 16), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Weiterhin können die erfindungsgemäßen Mittel als Fettstoff mindestens ein aminofunktionelles Silikon der Formel (Si3-a) enthalten, 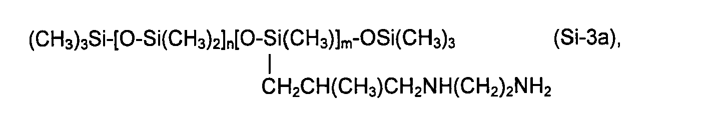 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Mittel, die als Fettstoff mindestens ein aminofunktionelles Silikon der Formel (Si-3b) 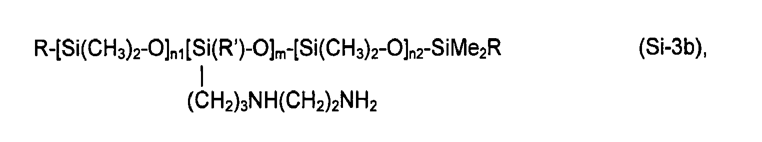 enthalten, worin
- R: für -OH, eine (gegebenenfalls ethoxylierte und/oder propoxylierte) (C₁ bis C₂₀)-Alkoxygruppe oder eine -CH₃-Gruppe steht,
- R': für -OH, eine (C₁ bis C₂₀)-Alkoxygruppe oder eine -CH₃-Gruppe und
- m, n1 und n2: Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich) bezeichnet.

Unabhängig davon, welche aminofunktionellen Silikone eingesetzt werden, sind erfindungsgemäße Mittel bevorzugt, die als Fettstoff ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silikons. Sie kann durch Titration ermittelt und auch in der Einheit mg KOH/g angegeben werden.

Erfindungsgemäß bevorzugte kosmetische oder dermatologische Zubereitungen sind dadurch gekennzeichnet, dass sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als Cyclomethicone bezeichneten cyclischen Dimethicone sind erfindungsgemäß mit Vorzug einsetzbar. Hier sind erfindungsgemäße kosmetische oder dermatologische Zubereitungen bevorzugt, die mindestens ein Silikon der Formel (Si-4) 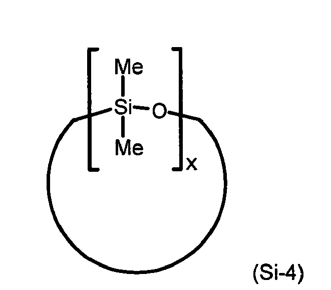 enthalten, in der x für eine Zahl von 0 bis 200, vorzugsweise von 0 bis 10, weiter bevorzugt von 0 bis 7 und insbesondere 0, 1, 2, 3, 4, 5 oder 6, steht.

Die vorstehend beschriebenen Silikone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Die erfindungsgemäß einsetzbaren Silikon-in-Wasser Emulsionen können nach bekannten Verfahren hergestellt werden, wie sie beispielsweise in US 5,998,537 und EP 0 874 017 A1 offenbart sind.

Zusammenfassend umfasst dieses Herstellungsverfahren die emulgierende Mischung von Komponenten, deren eine mindestens ein Polysiloxane enthält, deren andere mindestens ein Organosilikonmaterial enthält, das mit dem Polysiloxane in einer Kettenverlängerungsreaktion reagiert, wobei mindestens ein Metallion-enthaltender Katalysator für die Kettenverlängerungsreaktion, mindestens ein Tensid und Wasser zugegen sind.

Kettenverlängerungsreaktionen mit Polysiloxanen sind bekannt und können beispielsweise die Hydrosilylierungsreaktion umfassen, in der eine Si-H Gruppe mit einer aliphatisch ungesättigten Gruppe in Gegenwart eines Platin/Rhodium-Katalysators unter Bildung von Polysiloxanes mit einigen Si-(C)ₚ-Si Bindungen (p = 1-6) reagiert, wobei die Polysiloxane auch als Polysiloxane-Polysilalkylene-Copolymere bezeichnet werden.

Die Kettenverlängerungsreaktion kann auch die Reaktion einer Si-OH Gruppe (beispielsweise eines Hydroxy-terminierten Polysiloxans) mit einer Alkoxygruppe (beispielsweise Alkoxysilanen, Silikaten oder Alkoxysiloxanen) in Gegenwart eines metallhaltigen Katalysators unter Bildung von Polysiloxanen umfassen.

Die Polysiloxane, die in der Kettenverlängerungsreaktion eingesetzt werden, umfassen ein substantiell lineares Polymer der folgenden Struktur:

R-Si(R₂)-[-O-Si(R₂)-]ₙ-O-SiR₃

In dieser Struktur steht jedes R unabhängig voneinander für einen Kohlenwasserstoffrest mit bis zu 20 Kohlenstoffatomen, vorzugsweise mit 1 bis 6 C-Atomen, wie beispielsweise einer Alkylgruppe (beispielsweise Methyl, Ethyl, Propyl oder Butyl), eine Arylgruppe (beispielsweise Phenyl), oder die für die Kettenverlängerungsreaktion benötigte Gruppe ("reaktive Gruppe", beispielsweise Si-gebundene H-Atome, aliphatisch ungesättigte Gruppen wie Vinyl, Allyl oder Hexenyl, Hydroxy, Alkoxy wie Methoxy, Ethoxy oder Propoxy, Alkoxy-Alkoxy, Acetoxy, Amino usw.), mit der Maßgabe, dass durchschnittlich ein bis zwei reaktive Gruppen pro Polymer vorliegen, n ist eine positive Zahl > 1. Vorzugsweise ist eine Mehrzahl der reaktiven Gruppen, besonders bevorzugt > 90%, und insbesondere > 98% der reaktiven Gruppen, an den endständigen Si-Atomen im Siloxan gebunden. Vorzugsweise steht n für Zahlen, die Polysiloxane beschreiben, welche Viskositäten zwischen 1 und 1.000.000 mm²/s besitzen, besonders bevorzugt Viskositäten zwischen 1.000 und 100.000 mm²/s.

Die Polysiloxane können zu einem geringen Grad verzweigt sein (beispielsweise < 2 Mol-% der Siloxaneinheiten), bzw. sind die Polymere aber substantiell linear, besonders bevorzugt vollständig linear. Zudem können die Substituenten R ihrerseits substituiert sein, beispielsweise mit N-haltigen Gruppen (beispielsweise Aminogruppen), Epoxygruppen, S-haltige Gruppen, Sihaltige Gruppen, O-haltige Gruppen usw.. Vorzugsweise sind mindestens 80% der Reste R Alkylreste, besonders bevorzugt Methylgruppen.

Das Organosilikonmaterial, das mit dem Polysiloxan in der Kettenverlängerungsreaktion reagiert, kann entweder ein zweites Polysiloxan sein, oder ein Molekül, das als Kettenverlängerer agiert. Wenn das Organosilikonmaterial ein Polysiloxan ist, hat es die vorstehend erwähnte generelle Struktur. In diesen Fällen besitzt ein Polysiloxan in der Reaktion (mindestens) eine reaktive Gruppe, und ein zweites Polysiloxan besitzt (mindestens) eine zweite reaktive Gruppe, die mit der ersten reagiert.

Falls das Organosilikonmaterial ein Kettenverlängerungs-Agens umfasst, kann dies ein Material sein wie beispielsweise ein Silan, ein Siloxan (beispielsweise Disiloxane oder Trisiloxan) oder ein Silazan. So kann beispielsweise eine Zusammensetzung, die ein Polysiloxan gemäß der vorstehend beschriebenen generellen Struktur umfasst, welches mindestens eine Si-OH Gruppe aufweist, ketteverlängert werden, indem mit einem Alkoxysilan (beispielsweise einem Dialkoxysilan oder Trialkoxysilan) in Gegenwart von Zinn- oder Titan-haltigen Katalysatoren reagiert wird.

Die metallhaltigen Katalysatoren in der Kettenverlängerungsreaktion sind meist spezifisch für eine bestimmte Reaktion. Solche Katalysatoren sind im Stand der Technik bekannt und enthalten beispielsweise Metalle wie Platin, Rhodium, Zinn, Titan, Kupfer, Blei, etc.. In einer bevorzugten Kettenverlängerungsreaktion wird ein Polysiloxan mit mindestens einer aliphatisch ungesättigten Gruppe, vorzugsweise einer Endgruppe, mit einem Organosilikonmaterial in Gegenwart eines Hydrosilylierungskatalysators zur Reaktion gebracht, das ein Siloxan oder Polysiloxan mit mindestens einer (vorzugsweise endständigen) Si-H Gruppe ist. Das Polysiloxan besitzt mindestens eine aliphatisch ungesättigte Gruppe und genügt der allgemeinen oben angegeben Formel, in der R und n wie vorstehend definiert sind, wobei im Durchschnitt zwischen 1 und 2 Gruppen R eine aliphatisch ungesättigte Gruppe pro Polymer besitzen. Repräsentative aliphatisch ungesättigte Gruppen sind beispielsweise Vinyl, Allyl, Hexenyl und Cyclohexenyl oder eine Gruppe R²CH=CHR³, in der R² für eine divalente aliphatische an das Silicium gebundene Kette und R³ für ein Wasserstoffatom oder eine Alkylgruppe steht. Das Organosilikonmaterial mit mindestens einer Si-H Gruppe hat vorzugsweise die oben genannte Struktur, in der R und n wie vorstehend definiert sind und wobei im Durchschnitt zwischen 1 und 2 Gruppen R ein Wasserstoff bedeuten und n 0 oder eine positive ganze Zahl ist.

Dieses Material kann ein Polymer oder ein niedermolekulares Material wie ein Siloxan sein (beispielsweise ein Disiloxane oder ein Trisiloxan).

Das Polysiloxan mit mindestens einer aliphatisch ungesättigten Gruppe und das Organosilikonmaterial mit mindestens einer Si-H Gruppe reagieren in Gegenwart eines Hydrosilylierungskatalysators. Solche Katalysatoren sind aus dem Stand der Technik bekannt und umfassen beispielsweise Platin- und Rhodium-enthaltende Materialien. Die Katalysatoren können jede bekannte Form annehmen, beispielsweise auf Trägermaterialien (wie beispielsweise Silica Gel oder Aktivkohle) aufgebrachtes Platin oder Rhodium oder andere geeignete Compounds wie Platinchlorid, Salze von Platin- oder Chloroplatinsäuren. Ein wegen der guten Dispergierbarkeit in Organosilikonsystemen und der geringen Farbveränderung bevorzugter Katalysator ist Chloroplatinsäure entweder als kommerziell verfügbares Hexahydrat oder in wasserfreier Form.

Bei einer weiteren bevorzugten Kettenerweiterungsreaktion wird ein Polysiloxan mit mindestens einer Si-OH Gruppe, vorzugsweise einer Endgruppe, mit einem Organosilikonmaterial zu Reaktion gebracht, das mindestens eine Alkoxygruppe besitzt, vorzugsweise ein Siloxan mit mindestens einer Si-OR Gruppe oder ein Alkoxysilan mit mindestens zwei Alkoxygruppen. Hierbei wird als Katalysator wieder ein metallhaltiger Katalysator eingesetzt.

Für die Reaktion einer Si-OH Gruppe mit einer Si-OR Gruppe existieren viele literaturbekannte Katalysatoren, beispielsweise Organometallverbindungen wie Organozinnsalze, Titanate oder Titanchelate bzw. -komplexe. Beispiele umfassen Zinn-octoat, Dibutylzinn-dilaurat, Dibutylzinndiacetat, Dimethyltinn-dineodecanoat, Dibutylzinn-dimethoxid, Isobutylzinn-triceroat, Dimethylzinn-dibutyrat, Dimethylzinn-dineodecanoat, Triethylzinn-tartrat, Zinnoleat, Zinnnaphthenat, Zinnbutyrat, Zinnacetat, Zinnbenzoat, Zinnsebacat, Zinnsuccinat, Tetrabutyltitanat, Tetraisopropyltitante, Tetraphenyltitanat, Tetraoctadecyltitanat, Titannaphthanat, Ethyltriethanolamin-Titanat, Titani-diisopropyl-diethyl-acetoacetat, Titan-diisopropoxydiacetyl-acetonat und Titani-tetra-Alkoxide, bei denen das Alkoxid Butoxy oder Propoxy ist.

Erfindungsgemäß ebenfalls bevorzugte Mittel sind dadurch gekennzeichnet, dass sie als Fettstoff mindestens ein Silikon der Formel (Si-5)

R₃Si-[O-SiR₂]ₓ-(CH₂)ₙ[O-SiR₂]_{y}-O-SiR₃ (Si-5),

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mindestens eine Fettsäure wird erfindungsgemäß bevorzugt als Fettstoff in den Färbemitteln eingesetzt, wobei wiederum lineare und/oder verzweigte, gesättigte und/oder ungesättigte Fettsäuren mit 6 bis 30 Kohlenstoffatomen vorzuziehen sind. Besonders geeignet sind lineare und/oder verzweigte, gesättigte und/oder ungesättigte Fettsäuren mit 10 bis 22 Kohlenstoffatomen. Hierunter wären beispielsweise zu nennen die Isostearinsäure, wie die Handelsprodukte Emersol^{®} 871 und Emersol^{®} 875, und die Isopalmitinsäure wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure und/oder Isostearinsäure.

Die Einsatzmenge an Fettsäuren beträgt dabei bevorzugt 0,1 bis 15 Gew.%, bezogen auf das gesamte Mittel. In einer besonders bevorzugten Ausführungsform beträgt die Menge 0,5 bis 10 Gew.%, wobei ganz besonders vorteilhaft Mengen von 1 bis 5 Gew.% sind.

Als Fettalkohole werden als Fettstoff bevorzugt eingesetzt gesättigte, ein- oder mehrfach ungesättigte, verzweigte oder unverzweigte Fettalkohole mit mit 6 bis 30 Kohlenstoffatomen, bevorzugt mit 10 bis 22 Kohlenstoffatomen und ganz besonders bevorzugt mit 12 bis 22 Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden bevorzugt in Mengen von 0,1 bis 20 Gew.%, bezogen auf die gesamte Zubereitung, besonders bevorzugt in Mengen von 0,1 bis 10 Gew.% eingesetzt.

Als natürliche oder synthetische Wachse kann erfindungsgemäß bevorzugt mindestens ein Wachs aus der Gruppe eingesetzt werden, die gebildet wird aus festen Paraffinen oder Isoparaffinen, Carnaubawachs, Bienenwachs, Candelillawachs, Ozokerit, Ceresin, Walrat, Sonnenblumenwachs, Fruchtwachs wie beispielsweise Apfelwachs oder Citruswachs, Microwachse aus Polyethylen oder Polypropylen. Derartige Wachse sind beispielsweise erhältlich über die Fa. Kahl & Co., Trittau.

Zu den natürlichen und synthetischen kosmetischen Ölkörpern, welche als Fettstoff im Sinne der Erfindung Anwendung finden, sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentyiether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀-Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12 oder Cutina^{®} MD.

Es ist erfindungsgemäß, mindestens einen organischen UV-Absorber in dem erfindungsgemäßen Mittel zu formulieren, der in mindestens einen Fettstoff der Fettphase eingearbeitet ist. Wenn der Fettstoff einen Schmelzpunkt über Raumtemperatur hat, so ist es bevorzugt, zur Einarbeitung des organischen UV-Absorbers in den Fettstoff den Fettstoff unter Erwärmung zu schmelzen und unter Rühren den organischen UV-Absorber hinzuzufügen. Wenn der Fettstoff, in den der organische UV-Absorber eingearbeitet wird bei Raumtemperatur flüssig ist, so kann der organische UV-Absorber unter Rühren in den flüssigen Fettstoff ohne vorheriges Erhitzen eingearbeitet werden.

Unter dem Merkmal, dass sich mindestens ein organischer UV-Absorber in mindestens einem Fettstoff der Fettphase befindet, ist erfindungsgemäß zu verstehen, dass (bezogen auf die Menge dieses UV-Absorbers) mehr als 50 Gew.-% (besonders bevorzugt mehr als 75 Gew.-%, ganz besonders bevorzugt mehr als 90 Gew.-%) eines im Färbemittel enthaltenen organischen UV-Absorbers in mindestens einen Fettstoff der Fettphase eingearbeitet sind. Falls mehrere organische UV-Absorber in dem erfindungsgemäßen Mittel enthalten ist, ist es ausreichend, wenn dieses Merkmal durch wenigstens einen dieser organischen UV-Absorber erfüllt wird. Bevorzugt wird dieses Merkmal in der Weise erfüllt, dass (bezogen auf die Menge aller im erfindungsgemäßen Mittel befindlichen organischen UV-Absorber) mehr als 50 Gew.-% (besonders bevorzugt mehr als 75 Gew.-%, ganz besonders bevorzugt mehr als 90 Gew.-%) der im Färbemittel enthaltenen organischen UV-Absorber in mindestens einen Fettstoff der Fettphase eingearbeitet sind.

Der erfindungsgemäß einzusetzende organische UV-Absorber absorbiert Licht im Wellenlängenbereich von kleiner 400 nm und gibt die aufgenommene Energie in Form längerwelliger Strahlung, z.B. Wärme wieder ab. Der Fachmann kennt verschiedene Arten des UV-Lichts, nämlich UV-A-Strahlung (315-400 nm), UV-B-Strahlung (280-315nm) und UV-C-Strahlung (<280 nm). Es ist erfindungsgemäß bevorzugt, wenn die in dem erfindungsgemäßen Färbemittel enthaltenen organischen UV-Filter Strahlung im UV-A Wellenlängenbereich und/oder im UV-B-Wellenlängenbereich absorbieren. Es ist erfindungsgemäß besonders bevorzugt, wenn mindestens ein organischer UV-Absorber in dem erfindungsgemäßen Mittel enthalten ist, der als Breitband UV-Absorber sowohl UV-A-Strahlung als auch UV-B-Strahlung absorbiert.

Die in dem erfindungsgemäßen Mittel enthaltenen organischen UV-Absorber werden bevorzugt ausgewählt aus mindestens einem UV-Absorber aus der Gruppe, die gebildet wird, aus

- Benzotriazol-Derivaten,
- Benzophenon-Derivaten,
- Benzyliden-Derivaten des Camphers (insbesondere 3-Benzylidencampher und dessen Derivate, z.B. 3-(4-Methylbenzyliden)campher, 4-(2-Oxo-3-bornylidenmethyl)benzolsulfonsäure oder 2-Methyl-5-(2-oxo-3-bornyliden)sulfonsäure und deren Salze),
- Salicylsäureester-Derivaten (vorzugsweise Salicylsäure-2-ethylhexylester, Salicylsäure-4-isopropylben-zylester oder Salicylsäurehomomenthylester),
- Zimtsäureester-Derivaten (vorzugsweise 4-Methoxyzimtsäure-2-ethylhexylester, 4-Methoxyzimtsäurepropylester, 4-Methoxyzimtsäureisoamylester oder 2-Cyano-3-phenyl-zimtsäure-2-ethylhexylester (Octocrylene)),
- Benzoesäureester-Derivaten (insbesondere 4-Aminobenzoesäurederivate, vorzugsweise 4-(Dimethylamino)benzoesäure-2-ethylhexylester, 4-(Dimethylamino)benzoesäure-2-octylester oder 4-(Dimethylamino)benzoesäureamylester),
- Triazin-Derivaten, (vorzugsweise 2,4,6-Trianilino-(p-carbo-2'-ethyl-1 '-hexyloxy)-1 ,3,5-triazin oder Octyltriazon),
- Dibenzoylmethan-Derivaten, (insbesondere 4-tert-Butyl-4'methoxy-dibenzoylmethan)
- Benzoxazol-Derivaten und
- Benzimidazol-Derivaten (beispielsweise 2-Phenylbenzimidazol-5-sulfonsäure oder deren Alkali-, Erdalkali-, Ammonium-, Alkylammonium-, Alkanolammonium- oder Glucammoniumsalze).

Dabei sind öllösliche UV-Absorber erfindungsgemäß bevorzugt. Im Sinne der Erfindung öllösliche UV-Absorber lösen sich zu mehr als 1g in 100 g Paraffinum Liquidum bei Standardbedingungen (25°C, 101325 Pa).

Die organischen UV-Absorber sind bevorzugt in einer Menge von 0,05 bis 10,0 Gew.-%, insbesondere von 0,1 bis 5,0 Gew.-%, jeweils bezogen auf das Gewicht des Färbemittels, in dem erfindungsgemäßen Mittel enthalten.

Es ist besonders bevorzugt, wenn als organischer UV-Absorber mindestens eine Verbindung der Formel (I) enthalten ist, 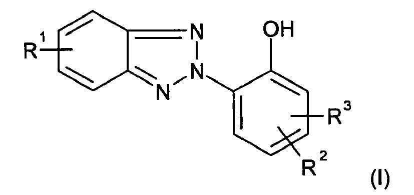 worin
- R¹: steht für ein Wasserstoffatom, ein Chloratom, ein Bromatom, eine Hydroxygruppe, eine (C₁ bis C₄)-Alkoxygruppe oder eine (C₁ bis C₄)-Alkylgruppe,
- R²: steht für eine lineare oder verzweigte (C₁ bis C₃₀)-Alkylgruppe, eine Arylgruppe, eine Aryl-(C₁ bis C₄)-alkylgruppe, eine (C₅ bis C₇)-Cycloalkylgruppe, eine (C₁ bis C₄)-Alkoxycarbonylgruppe, eine Sulfonylgruppe, eine Sulfonatgruppe oder eine Sulfonsäureester-Gruppe,
- R³: steht für eine (C₁ bis C₃₀)-Alkylgruppe, eine lineare oder verzweigte (C₁ bis C₃₀)-Acyloxygruppe eine (C₈ bis C₂₂)-Alkenylgruppe oder eine Hydroxybenzylgruppe der Formel (11), 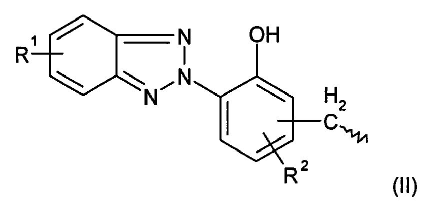 worin R¹ und R² wie oben definiert sind.

Dabei ist es wiederum besonders bevorzugt, wenn die Benzotriazol-Derivate ausgewählt werden unter Derivaten der Formel (I-1) 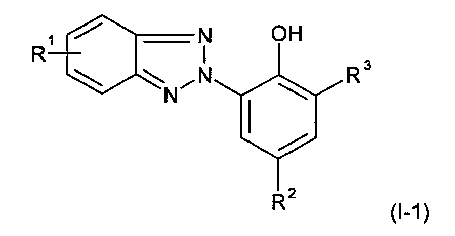 worin
- R¹: steht für ein Wasserstoffatom, ein Chloratom, ein Bromatom, eine Hydroxygruppe, eine (C₁ bis C₄)-Alkoxygruppe oder eine (C₁ bis C₄)-Alkylgruppe,
- R²: steht für eine lineare oder verzweigte (C₁ bis C₃₀)-Alkylgruppe, eine Arylgruppe, eine Aryl-(C₁ bis C₄)-alkylgruppe, eine (C₅ bis C₇)-Cycloalkylgruppe, eine (C₁ bis C₄)-Alkoxycarbonylgruppe, eine Sulfonylgruppe oder eine Sulfonatgruppe,
- R³: steht für eine (C₁ bis C₃₀)-Alkylgruppe, eine lineare oder verzweigte (C₁ bis C₃₀)-Acyloxygruppe eine (C₈ bis C₂₂)-Alkenylgruppe oder eine Hydroxybenzylgruppe der Formel (II-1), 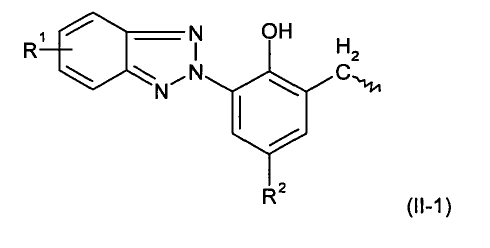 worin R¹ und R² wie oben definiert sind.

Benzotriazolderivate gemäß Formel (I), die öllöslich gemäß obiger Definition sind und sich daher in mindestens einen Fettstoff der Fettphase des erfindungsgemäßen Mittels einarbeiten lassen, sind erfindungsgemäß bevorzugte Benzotriazol-Derivate.

Gemäß Formel (I) bzw. Formel (I-1) ist es erfindungsgemäß bevorzugt, wenn die (C₁ bis C₃₀)-Alkylgruppen ausgewählt werden aus Methyl, Ethyl, n-Propyl, Isopropyl, n-Butyl, sec-Butyl, tert-Butyl, n-Pentyl, Isopentyl, tert-Pentyl, Neopentyl, Hexyl, 2-Ethylhexyl, Heptyl, n-Octyl, Isooctyl, Nonyl, Decyl, Undecyl, Dodecyl, Tetradecyl, Pentadecyl, Hexadecyl, Heptadecyl, Octadecyl oder Eicosanyl.

Gemäß Formel (I) bzw. Formel (I-1) ist es erfindungsgemäß bevorzugt, wenn die (C₁ bis C₄)-Alkylgruppen ausgewählt werden aus Methyl, Ethyl, n-Propyl, Isopropyl, n-Butyl oder sec-Butyl, tert-Butyl.

Gemäß Formel (I) bzw. Formel (I-1) ist es erfindungsgemäß bevorzugt, wenn die (C₅ bis C₇)-Cycloalkylgruppen ausgewählt werden aus Cyclopentyl, Cyclohexyl oder Cycloheptyl, insbesondere Cyclohexyl.

Gemäß Formel (I) bzw. Formel (I-1) ist es erfindungsgemäß bevorzugt, wenn die Arylgruppen ausgewählt werden aus Naphthyl oder Phenyl, insbesondere Phenyl.

Gemäß Formel (I) bzw. Formel (I-1) ist es erfindungsgemäß bevorzugt, wenn die Aryl-(C₁ bis C₄)-alkylgruppen ausgewählt werden aus 2-Phenylethyl, 2-Phenylprop-2-yl oder Benzyl.

Gemäß Formel (I) bzw. Formel (I-1) ist es erfindungsgemäß bevorzugt, wenn R¹ für ein Wasserstoffatom steht.

Gemäß Formel (I) bzw. Formel (I-1) ist es erfindungsgemäß bevorzugt, wenn R² steht für eine lineare oder verzweigte (C₁ bis C₃₀)-Alkylgruppe, eine Arylgruppe, eine Aryl-(C₁ bis C₄)-alkylgruppe, eine (C₅ bis C₇)-Cycloalkylgruppe, eine (C₁ bis C₄)-Alkoxycarbonylgruppe.

Ganz besonders bevorzugt wird als Benzotriazolderivat mindestens eine Verbindung der Formel (I-2) ausgewählt, 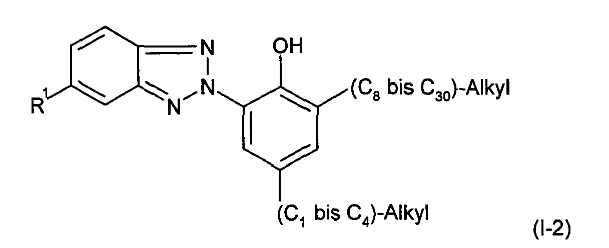 worin
- R¹: steht für ein Wasserstoffatom oder ein Chloratom.

Gemäß Formel (1-2) ist es bevorzugt, wenn R¹ für ein Wasserstoffatom steht. Dabei ist es wiederum bevorzugt, wenn die (C₈ bis C₃₀)-Alkylgruppe linear und/oder verzweigt ist. Ganz besonders bevorzugt stellt der organische UV-Absorber mindestens ein Stoffgemisch von Verbindungen der Formel (1-2) dar, in dem die (C₈ bis C₃₀)-Alkylgruppe linear und verzweigt ist (insbesondere eine lineare und eine verzweigte C₁₂H₂₅-Gruppe und/oder eine lineare und eine verzweigte C₁₄H₂₉-Gruppe und/oder eine lineare und eine verzweigte C₁₆H₃₃-Grupp bedeutet).
Die (C₁ bis C₄)-Alkylgruppe der Formel (1-2) steht bevorzugt für eine Methylgruppe.

Bevorzugte organische UV-Absorber der Formel (1) werden ausgewählt aus mindestens einer Verbindung der Gruppe, die gebildet wird, aus 2-(2H-Benzotriazol-2-yl)-4-methyl-5-dodecylphenol, 2-(2H-Benzotriazol-2-yl)-4-methyl-6-dodecylphenol, 2-(2H-Benzotriazol-2-yl)-4-tert-butylphenol, 2-(5-Chloro-2H-benzotriazol-2-yl)-6-tert-butyl-4-methylphenol, 2,2'-Methylen-bis(6-(2H-benzotriazol-2-yl)-4-(1,1,3,3-tetramethylbutyl)phenol), 2-(2H-Benzotriazol-2-yl)-4-methylphenol, 2-(5-Chloro-2H-benzotriazol-2-yl)-4,6-di(tert-butyl)phenol, 2-(2H-Benzotriazol-2-yl)-4-methyl-6-C₁₂H₂₅-phenol, Natrium 3-(2H-Benzotriazol-2-yl)-4-hydroxy-5-(sec-butyl)benzolsulfonat, 2-(2H-Benzotriazol-2-yl)-4-tert-butyl-6-sec-butylphenol, 2-(2H-Benzotriazol-2-yl)-4-(1,1,3,3-tetramethylbutyl)-phenolsulfonsäureester, 2-(2H-Benzotriazol-2-yl)-4-(1,1,3,3-tetramethylbutyl)phenol, 2-(2H-Benzotriazol-2-yl)-4,6 bis(1,1,3,3-tetramethylbutyl)phenol, 2-(2H-Benzotriazol-2-yl)-4,6 bis(tert-pentyl)phenol und 2-(2H-Benzotriazol-2-yl)-4,6-bis(2-phenylprop-2-yl)phenol.

Als besonders bevorzugter UV-Absorber gemäß Formel (I) bzw. (I-1) bzw. (I-2) ist der unter dem Handelsnamen Tinogard^{®} TL (INCI-Bezeichnung: Benzotriazolyl Dodecyl p-Cresol; einem Stoffgemisch aus Verbindungen der Formel (I-2), worin die (C₈ bis C₃₀)-Alkylgruppe der Formel (I-2) für eine lineare und für eine verzweigte C₁₂H₂₅-Gruppe steht und die (C₁ bis C₄)-Alkylgruppe der Formel (I-2) eine Methylgruppe bedeutet) von der Fa. Clariant vermarktete UV-Absorber zu nennen.

Insbesondere sind organische UV-Absorber der Formeln (1), (1-1) bzw. (1-2) bevorzugt, die öllöslich sind, wobei dabei wiederum solche bevorzugt sind, die bei Standardbedingungen (25°C, 101325 Pa) flüssig sind.

Der UV-Absorber gemäß Formel (I) bzw. Formel (I-1) bzw. Formel (I-2) ist bevorzugt in einer Menge von 0,01 bis 8 Gew.-% insbesondere von 0,05 bis 5 Gew.%, jeweils bezogen auf das Gewicht des Färbemittels, in dem erfindungsgemäßen Mittel enthalten.

Weiterhin ist es erfindungsgemäß bevorzugt als organischen UV-Absorber mindestens ein Derivat des Benzophenons gemäß Formel (III) einzusetzen, 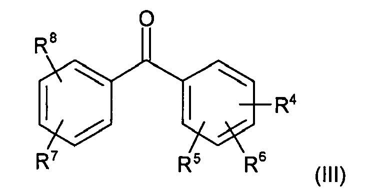 worin
R⁴, R⁵, R⁶, R⁷ und R⁸ unabhängig voneinander stehen für ein Wasserstoffatom, ein Halogenatom (insbesondere ein Chloratom), eine Hydroxygruppe, eine (C₁ bis C₁₀)-Alkyloxygruppe, eine Sulfonsäuregruppe oder eine Sulfonatgruppe, mit der Maßgabe, dass mindestens ein Rest aus R⁴, R⁵, R⁶, R⁷ und R⁸ von einem Wasserstoffatom verschieden ist.

Die organischen UV-Absorber der Formel (III) werden bevorzugt ausgewählt aus mindestens einer Verbindung aus der Gruppe, die gebildet wird, aus 2,4-Dihydroxybenzophenone (Benzophenone 1), 2,2',4,4'-Tetrahydroxybenzophenon (Benzophenone 2), 2-Hydroxy-4-(methoxy)benzophenon (Benzophenone 3), 2-Hydroxy-4-methoxybenzophenon-5-sulfonsäure (Benzophenone 4) bzw. ein Salz davon, 2,2'-Dihydroxy-4,4'-dimethoxybenzophenon (Benzophenone 6), 5-Chlor-2-hydroxybenzophenon (Benzophenone 7), 2,2'-Dihydroxy-4-methoxybenzophenon, 2,2'-Dihydroxy-4-octyloxybenzophenon, 2-Hydroxy-4-(octyloxy)benzophenon (Benzophenone 12). Besonders bevorzugt enthält das erfindungsgemäße Färbemittel als organischen UV-Absorber 2-Hydroxy-4-(methoxy)benzophenon (Benzophenone 3) und/oder 2-Hydroxy-4-methoxybenzophenon-5-sulfonsäure (Benzophenone 4) oder ein Salz davon.

Es ist erfindungsgemäß bevorzugt, die organischen UV-Absorber der Formel (III) in einer Menge von 0,5 Gew.% bis 10 Gew.%, insbesondere von 1,0 Gew.% bis 8,0 Gew.%, jeweils bezogen auf das Gewicht des Mittels, enthalten.

Gemäß Erfindung wird ein besonders guter Effekt erhalten, wenn als organische UV-Absorber mindestens eine Verbindung der Formel (1) und mindestens eine Verbindung der Formel (111) gemeinsam in dem Mittel vorliegen. Dabei ist es wiederum bevorzugt mindestens einen organischen UV-Absorber gemäß Formel (I-2) mit mindestens einem organischen UV-Absorber der Formel (III) (insbesondere mit 2-Hydroxy-4-(methoxy)benzophenon (Benzophenone 3) und/oder 2-Hydroxy-4-methoxybenzophenon-5-sulfonsäure (Benzophenone 4)) zu kombinieren.
Im Rahmen dieser Kombination erwies es sich als vorteilhaft, wenn sich entweder beide organischen UV-Absorbertypen in mindestens einem Fettstoff der Fettphase befinden oder sich mindestens die organischen UV-Absorber der Formel (I) in mindestens einem Fettstoff der Fettphase befinden und die organischen UV-Absorber der Formel (III) in der wässrigen Phase.
Das Molverhältnis der Verbindungen der Formel (I) zu den Verbindungen der Formel (III) liegt bevorzugt im Bereich von 5 zu 1 bis 1 zu 100, besonders bevorzugt im Bereich von 2 zu 1 bis 1 zu 50.

Neben den genannten organischen UV-Absorbern kommen als zusätzlich in dem erfindungsgemäßen Mittel enthaltene UV-Absorber auch unlösliche anorganische Pigmente in Frage, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen.

Die farbverändernde Komponente wird bevorzugt ausgewählt
(1) aus mindestens einem Oxidationsfarbstoffvorprodukt vom Typ der Entwicklerkomponenten und gegebenenfalls zusätzlich mindestens einer Kupplerkomponente
   und/oder
(2) aus Oxofarbstoffvorprodukten
   und/oder
(3) aus mindestens einem direktziehenden Farbstoff
   und/oder
(4) aus mindestens einer Vorstufe naturanaloger Farbstoffe.

Es kann erfindungsgemäß bevorzugt sein, die erfindungsgemäßen Entwicklerkomponenten aus der Gruppe auszuwählen, die gebildet wird aus p-Phenylendiaminderivaten, zweikernigen Entwicklerkomponenten, p-Aminophenol und seinen Derivaten, Pyrimidinderivaten, Pyrazolderivaten sowie Pyrazozlopyrimidinderivaten und den physiologisch verträglichen Salzen dieser Verbindungen. Im Folgenden werden erfindungsgemäß bevorzugte Entwicklerkomponenten genannt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 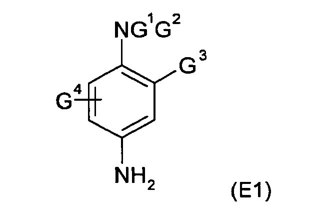 wobei
- G¹ steht für ein Wasserstoffatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen 4'-Aminophenylrest oder einen (C₁ bis C₄)-Alkylrest, der mit einer stickstoffhaltigen Gruppe, einem Phenyl- oder einem 4'-Aminophenylrest substituiert ist;
- G² steht für ein Wasserstoffatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest oder einen (C₁ bis C₄)-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Hydroxyalkoxyrest, einen (C₁ bis C₄)-Acetylaminoalkoxyrest, einen Mesylamino-(C₁ bis C₄)-alkoxyrest oder einen (C₁ bis C₄)-Carbamoylaminoalkoxyrest;
- G⁴ steht für ein Wasserstoffatom, ein Halogenatom oder einen (C₁ bis C₄)-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1 H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(β-Hydroxyethyl)-p-phenylendiamin, 2-(a,β-Dihydroxyethyl)-p-phenylendiamin, N,N-Bis-(β-hydroxyethyl)-p-phenylendiamin, N-(4-Amino-3-methylphenyl)-N-[3-(1 H-imidazol-1-yl)propyl]amin, sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 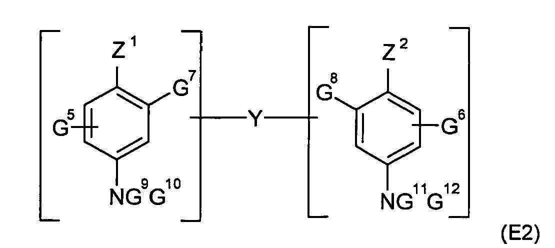 wobei:
- Z¹ und Z² stehen unabhängig voneinander für einen Hydroxyl- oder NH₂-Rest, der gegebenenfalls durch einen (C₁ bis C₄)-Alkylrest, durch einen (C₁ bis C₄)-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder (C₁ bis C₈)-Alkoxyreste substituiert sein kann, oder eine direkte Bindung,
- G⁵ und G⁶ stehen unabhängig voneinander für ein Wasserstoff- oder Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen (C₁ bis C₄)-Alkylrest,
mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) werden insbesondere aus mindestens einer der folgenden Verbindungen ausgewählt: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)phenyl)-tetra-methylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Ganz besonders bevorzugte zweikernige Entwicklerkomponenten der Formel (E2) werden ausgewählt unter N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 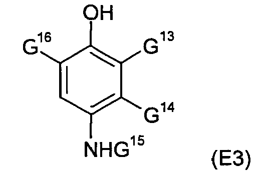 wobei:
- G¹³ steht für ein Wasserstoffatom, ein Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen (C₁ bis C₄)-Aminoalkylrest, einen Hydroxy-(C₁ bis C₄)-alkylaminorest, einen (C₁ bis C₄)-Hydroxyalkoxyrest, einen (C₁ bis C₄)-Hydroxyalkyl-(C₁ bis C₄)-aminoalkylrest oder einen (Di-[(C₁ bis C₄)-alkyl]amino)-(C₁ bis C₄)-alkylrest, und
- G¹⁴ steht für ein Wasserstoff- oder Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen (C₁ bis C₄)-Aminoalkylrest oder einen (C₁ bis C₄)-Cyanoalkylrest,
- G¹⁵ steht für Wasserstoff, einen (C₁ bis C₄)-Alkylrest, einen (C₁ bis C₄)-Monohydroxyalkylrest, einen (C₂ bis C₄)-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(a,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Bevorzugte Pyrimidin-Derivate werden erfindungsgemäß ausgewählt aus Verbindungen gemäß Formel (E4) bzw. deren physiologisch verträglichen Salzen, 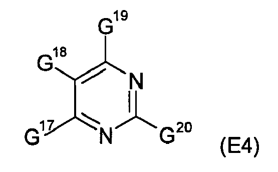 worin
- G¹⁷ , G¹⁸ und G¹⁹ unabhängig voneinander für ein Wasserstoffatom, eine Hydroxygruppe, eine (C₁ bis C₄)-Alkoxygruppe oder eine Aminogruppe steht und
- G²⁰ für eine Hydroxygruppe oder eine Gruppe -NG²¹G²² steht, worin G²¹ und G²² unabhängig voneinander stehen für ein Wasserstoffatom, eine (C₁ bis C₄)-Alkylgruppe, eine (C₁ bis C₄)-Monohydroxyalkylgruppe,
mit der Maßgabe, dass maximal zwei der Gruppen aus G¹⁷ , G¹⁸, G¹⁹ und G²⁰ eine Hydroxygruppe bedeuten und höchstens zwei der Reste G¹⁷ , G1B und G¹⁹ für ein Wasserstoffatom stehen. Dabei ist es wiederum bevorzugt, wenn gemäß Formel (E4) mindestens zwei Gruppen aus G¹⁷, G¹⁸, G¹⁹ und G²⁰ für eine Gruppe -NG²¹G²² stehen und höchstens zwei Gruppen aus G¹⁷ , G¹⁸, G¹⁹ und G²⁰ für eine Hydroxygruppe stehen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate werden erfindungsgemäß ausgewählt aus Verbindungen gemäß Formel (E5), 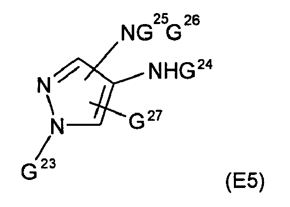 worin
- G²³, G²⁴, G²⁵ stehen unabhängig voneinander für ein Wasserstoffatom, eine (C₁ bis C₄)-Alkylgruppe, eine (C₁ bis C₄)-Monohydroxyalkylgruppe, eine (C₂ bis C₄)-Polyhydroxyalkylgruppe, eine gegebenenfalls substituierte Arylgruppe oder eine gegebenenfalls substituierte Aryl-(C₁ bis C₄)-alkylgruppe, mit der Maßgabe dass, wenn G²⁵ für ein Wasserstoffatom steht, G²⁶ neben den vorgenannten Gruppen zusätzlich für eine Gruppe - NH₂ stehen kann,
- G²⁶ steht für ein Wasserstoffatom, eine (C₁ bis C₄)-Alkylgruppe, eine (C₁ bis C₄)-Monohydroxyalkylgruppe oder eine (C₂ bis C₄)-Polyhydroxyalkylgruppe und
- G²⁷ steht für ein Wasserstoffatom, eine gegebenenfalls substituierte Arylgruppe, eine (C₁ bis C₄)-Alkylgruppe oder eine (C₁ bis C₄)-Monohydroxyalkylgruppe, insbesondere für ein Wasserstoffatom oder eine Methylgruppe.

Bevorzugt bindet in Formel (E5) der Rest -NG²⁵G²⁶ an die 5 Position und der Rest G²⁷ an die 3 Position des Pyrazolzyklus.

Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methyl-pyrazol, 4,5-Diamino-3-hydroxymethyl-1-isopropylpyrazol, 4,5-Diamino-3-methyl-1-isopropyl-pyrazol, 4-Amino-5-(β-aminoethyl)amino-1,3-dimethylpyrazol, sowie deren physiologisch verträglichen Salze.

Bevorzugte Pyrazolopyrimidin-Derivate sind insbesondere die Derivate des Pyrazolo[1,5-a]pyrimidin der folgenden Formel (E6) und dessen tautomeren Formen, sofern ein tautomeres Gleichgewicht besteht: 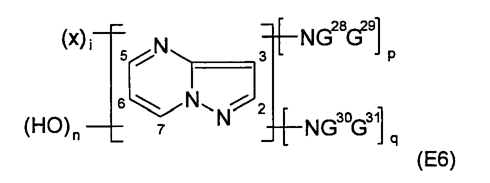 wobei:
- G²⁸, G²⁹ und G³⁰, G³¹unabhängig voneinander stehen für ein Wasserstoffatom, einen (C₁ bis C₄)-Alkylrest, einen Aryl-Rest, einen (C₁ bis C₄)-Monohydroxyalkylrest, einen (C₂ bis C₄)-Polyhydroxyalkylrest einen (C₁ bis C₄)-Alkoxy-(C₁ bis C₄)-alkylrest, einen (C₁ bis C₄)-Aminoalkylrest, der gegebenenfalls durch ein Acetyl-Ureid- oder einen Sulfonyl-Rest geschützt sein kann, einen (C₁ bis C₄)-Alkylamino-(C₁ bis C₄)-alkylrest, einen Di-[(C₁ bis C₄)-alkyl]-(C₁ bis C₄)-aminoalkylrest, wobei die Dialkyl-Reste gegebenenfalls einen Kohlenstoffzyklus oder einen Heterozyklus mit 5 oder 6 Kettengliedern bilden, einen (C₁ bis C₄)-Monohydroxyalkyl- oder einen Di[(C₁ bis C₄)-Hydroxyalkyl]-(C₁ bis C₄)-aminoalkylrest,
- die X-Reste stehen unabhängig voneinander für ein Wasserstoffatom, einen (C₁ bis C₄)-Alkylrest, einen Aryl-Rest, einen (C₁ bis C₄)-Monohydroxyalkylrest, einen (C₂ bis C₄)-Polyhydroxyalkylrest, einen (C₁ bis C₄)-Aminoalkylrest, einen (C₁ bis C₄)-Alkylamino-(C₁ bis C₄)-alkylrest, einen Di-[(C₁ bis C₄)alkyl]-(C₁ bis C₄)-aminoalkylrest, wobei die Dialkyl-Reste gegebenenfalls einen Kohlenstoffzyklus oder einen Heterozyklus mit 5 oder 6 Kettengliedern bilden, einen (C₁ bis C₄)-Hydroxyalkyl- oder einen Di-[(C₁ bis C₄)-hydroxyalkyl]amino-(C₁ bis C₄)-alkylrest, einen Aminorest, einen (C₁ bis C₄)-Alkyl- oder Di-[(C₁ bis C₄)-hydroxyalkyl]aminorest, ein Halogenatom, eine Carboxylsäuregruppe oder eine Sulfonsäuregruppe,
- i hat den Wert 0,1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG²⁸G²⁹ und NG³⁰G³¹ belegen die Positionen (2,3); (5,6); (6,7); (3,5) oder (3,7);
- wenn p + q gleich 1 ist, n den Wert 1 hat, und die Gruppen NG²⁸G²⁹ (oder NG³⁰G³¹) und die Gruppe OH belegen die Positionen (2,3); (5,6); (6,7); (3,5) oder (3,7);

Die in Formel (E7) verwendeten Substituenten sind erfindungsgemäß analog zu den obigen Ausführungen definiert.

Wenn das Pyrazolo[1,5-a]pyrimidin der obenstehenden Formel (E6) eine Hydroxygruppe an einer der Positionen 2, 5 oder 7 des Ringsystems enthält, besteht ein tautomeres Gleichgewicht, das zum Beispiel im folgenden Schema dargestellt wird:

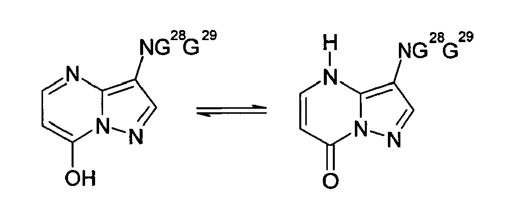

Unter den Pyrazolo[1,5-a]pyrimidinen der obenstehenden Formel (E7) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)-amino]-ethanol;
- 2-[(7-Aminopyrazolo[1,5-a]pyrimidin-3-yl)-(2-hydroxy-ethyl)-amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomers Gleichgewicht vorhanden ist.

Die Pyrazolo[1,5-a]pyrimidine der obenstehenden Formel (E6) können wie in der Literatur beschrieben durch Zyklisierung ausgehend von einem Aminopyrazol oder von Hydrazin hergestellt werden.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Im folgenden werden Beispiele für die als Substituenten der Verbindungen der Formeln (E1) bis (E6) genannten Reste aufgezählt: Beispiele für (C₁ bis C₄)-Alkylreste sind die Gruppen -CH₃, - CH₂CH₃,
-CH₂CH₂CH₃, -CH(CH₃)₂, -CH₂CH₂CH₂CH₃, -CH₂CH(CH₃)₂, -CH(CH₃)CH₂CH₃, -C(CH₃)₃. Erfindungsgemäße Beispiele für (C₁ bis C₄)-Alkoxyreste sind -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, - OC(CH₃)₃, insbesondere eine Methoxy- oder eine Ethoxygruppe.
Weiterhin können als bevorzugte Beispiele für eine (C₁ bis C₄)-Monohydroxyalkylgruppe -CH₂OH, -CH₂CH₂OH, -CH₂CH₂CH₂OH, -CHCH(OH)CH₃, -CH₂CH₂CH₂CH₂OH wobei die Gruppe - CH₂CH₂O bevorzugt ist.
Ein besonders bevorzugtes Beispiel einer (C₂ bis C₄)-Polyhydroxyalkylgruppe ist die 1,2-Dihydroxyethylgruppe.
Beispiele für Halogenatome sind F-, Cl- oder Br-Atome, Cl-Atome sind ganz besonders bevorzugte Beispiele.
Beispiele für stickstoffhaltige Gruppen sind insbesondere -NH₂, (C₁ bis C₄)-Monoalkylaminogruppen, (C₁ bis C₄)-Dialkylaminogruppen, (C₁ bis C₄)-Trialkylammoniumgruppen, (C₁ bis C₄)-Monohydroxyalkylaminogruppen, Imidazolinium und - NH₃⁺.
Beispiele für (C₁ bis C₄)-Monoalkylaminogruppen sind -NHCH₃, -NHCH₂CH₃, -NHCH₂CH₂CH₃, -NHCH(CH₃)₂.
Beispiele für (C₁ bis C₄)-Dialkylaminogruppe sind -N(CH₃)₂, -N(CH₂CH₃)₂.
Beispiele für (C₁ bis C₄)-Trialkylammoniumgruppen sind -N⁺(CH₃)₃, -N⁺(CH₃)₂(CH₂CH₃), -N⁺(CH₃)(CH₂CH₃)₂.
Beispiele für (C₁ bis C₄)-Hydroxyalkylaminoreste sind -NH-CH₂CH₂OH -NH-CH₂CH₂OH -NH-CH₂CH₂CH₂OH, -NH-CH₂CH₂CH₂OH.
Beispiele für (C₁ bis C₄)-Alkoxy-(C₁ bis C₄)-alkylgruppen sind die Gruppen -CH₂CH₂-O-CH₃, -CH₂CH₂CH₂-O-CH₃, -CH₂CH₂-O-CH₂CH₃, -CH₂CH₂CH₂-O-CH₂CH₃, -CH₂CH₂-O-CH(CH₃), -CH₂CH₂CH₂-O-CH(CH₃).
Beispiele für Hydroxy-(C₁ bis C₄)-alkoxyreste sind -O-CH₂OH, -O-CH₂CH₂OH, -O-CH₂CH₂CH₂OH, -O-CHCH(OH)CH₃, -O-CH₂CH₂CH₂CH₂OH.
Beispiele für (C₁ bis C₄)-Acetylaminoalkoxyreste sind -O-CH₂NHC(O)CH₃, -O-CH₂CH₂NHC(O)CH₃, -O-CH₂CH₂CH₂NHC(O)CH₃, -O-CH₂CH(NHC(O)CH₃)CH₃, -O-CH₂CH₂CH₂CH₂NHC(O)CH₃.
Beispiele für (C₁ bis C₄)-Carbamoylaminoalkoxyreste sind -O-CH₂CH₂-NH-C(O)-NH₂, -O-CH₂CH₂CH₂-N H-C(O)-NH_{2'} -O-CH₂CH₂CH₂CH₂-NH-C(O)-NH₂.
Beispiele für (C₁ bis C₄)-Aminoalkylreste sind -CH₂NH₂, -CH₂CH₂NH₂, -CH₂CH₂CH₂NH₂, -CH₂CH(NH₂)CH₃, -CH₂CH₂CH₂CH₂NH₂.
Beispiele für (C₁ bis C₄)-Cyanoalkylreste sind -CH₂CN, -CH₂CH₂CN, -CH₂CH₂CH₂CN.
Beispiele für (C₁ bis C₄)-Hydroxyalkylamino-(C₁ bis C₄)-alkylreste sind -CH₂CH₂NH-CH₂CH₂OH, -CH₂CH₂CH₂NH-CH₂CH₂OH, -CH₂CH₂NH-CH₂CH₂CH₂OH, -CH₂CH₂CH₂NH-CH₂CH₂CH₂OH.
Beispiele für Di[(C₁ bis C₄)-Hydroxyalkyl]amino-(C₁ bis C₄)-alkylreste sind - CH₂CH₂N(CH₂CH₂OH)₂, -CH₂CH₂CH₂N(CH₂CH₂OH)₂, -CH₂CH₂N(CH₂CH₂CH₂OH)₂, -CH₂CH₂CH₂N(CH₂CH₂CH₂OH)₂.
Ein Beispiel für Arylgruppen ist die Phenylgruppe.
Beispiele für Aryl-(C₁ bis C₄)-alkylgruppen sind die Benzylgruppe und die 2-Phenylethylgruppe.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,
Gemische aus zwei oder mehrer Verbindungen aus einer oder mehrerer dieser Klassen sind im Rahmen dieser Ausführungsform ebenso erfindungsgemäß.

Die erfindungsgemäß verwendbaren m-Aminophenole bzw. deren Derivate werden bevorzugt ausgewählt aus mindestens einer Verbindung der Formel (K1) und/oder aus mindestens einem physiologisch verträglichen Salz einer Verbindung gemäß Formel (K1), 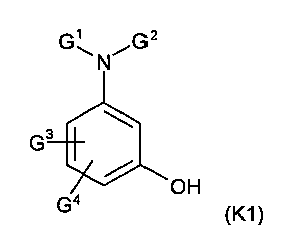 worin
- G¹ und G²: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₂ bis C₄)-Perfluoracylgruppe, eine Aryl-(C₁ bis C₆)-alkylgruppe, eine Amino-(C₁ bis C₆)-alkylgruppe, eine (C₁ bis C₆)-Dialkylamino-(C₁ bis C₆)-alkylgruppe oder eine (C₁ bis C₆)-Alkoxy-(C₁ bis C₆)-alkylgruppe, wobei G¹ und G² gemeinsam mit dem Stickstoffatom einen fünfgliedrigen, sechsgliedrigen oder siebengliedrigen Ring bilden können,
- G³ und G⁴: unabhängig voneinander stehen für ein Wasserstoffatom, ein Halogenatom, eine (C₁ bis C₄)-Alkylgruppe, eine (C₁ bis C₄)-Alkoxygruppe, eine Hydroxygruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe, eine (C₁ bis C₆)-Alkyox-(C₂ bis C₆)-alkoxygruppe, eine Arylgruppe oder eine Heteroarylgruppe.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4.-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Die erfindungsgemäß verwendbaren m-Diaminobenzole bzw. deren Derivate werden bevorzugt ausgewählt aus mindestens einer Verbindung der Formel (K2) und/oder aus mindestens einem physiologisch verträglichen Salz einer Verbindung gemäß Formel (K2), 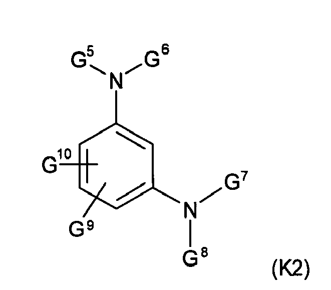 worin
- G⁵, G⁶, G⁷ und G⁸: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe, eine (C₂ bis C₄)-Perfluoracylgruppe, oder gemeinsam mit dem Stickstoffatom einen fünfgliedrigen oder sechsgliedrigen Heterozyklus bilden
- G⁹ und G¹⁰: unabhängig voneinander stehen für ein Wasserstoffatom, ein Halogenatom, eine (C₁ bis C₄)-Alkylgruppe, eine ω-(2,4-Diaminophenyl)-(C₁ bis C₄)-alkylgruppe, eine ω-(2,4-Diaminophenyloxy)-(C₁ bis C₄)-alkoxygruppe, eine (C₁ bis C₄)-Alkoxygruppe, eine Hydroxygruppe, eine (C₁ bis C₄)-Alkoxy-(C₂ bis C₄)-alkoxygruppe, eine Arylgruppe, eine Heteroarylgruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und den physiologisch verträglichen Salzen aller vorstehend genannten Verbindungen.

Die erfindungsgemäß verwendbaren o-Diaminobenzole bzw. deren Derivate werden bevorzugt ausgewählt aus mindestens einer Verbindung der Formel (K3) und/oder aus mindestens einem physiologisch verträglichen Salz einer Verbindung gemäß Formel (K3), 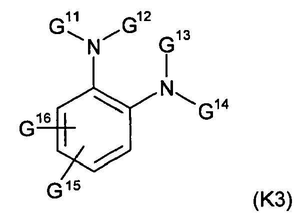 worin
- G¹¹, G¹², G¹³ und G¹⁴: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe, eine (C₂ bis C₄)-Perfluoracylgruppe, oder gemeinsam mit dem Stickstoffatom einen fünfgliedrigen oder sechsgliedrigen Heterozyklus bilden
- G¹⁵ und G¹⁶: unabhängig voneinander stehen für ein Wasserstoffatom, ein Halogenatom, eine Carboxylgruppe, eine (C₁ bis C₄)-Alkylgruppe, eine (C₁ bis C₄)-Alkoxygruppe, eine Hydroxygruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Formel (K4) und/oder aus mindestens einem physiologisch verträglichen Salz einer Verbindung gemäß Formel (K4), 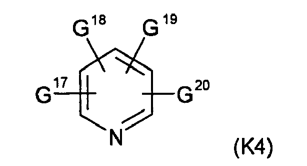 worin
- G¹⁷ und G¹⁸: stehen unabhängig voneinander für eine Hydroxygruppe oder eine Gruppe - NG²¹G²²,
worin G²¹ und G²² unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine Arylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe,
- G¹⁹ und G²⁰: stehen unabhängig voneinander für ein Wasserstoffatom, ein Halogenatom, eine (C₁ bis C₄)-Alkylgruppe oder eine (C₁ bis C₄)-Alkoxygruppe.

Es ist bevorzugt, wenn gemäß Formel (K4) die Reste G¹⁷ und G¹⁸ in ortho-Position oder in meta-Position zueinander stehen.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus mindestens einer Verbindung der Formel (K5) und/oder aus mindestens einem physiologisch verträglichen Salz einer Verbindung gemäß Formel (K5), 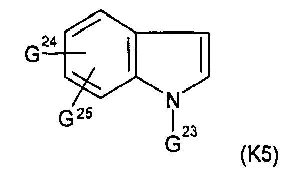 worin
- G²³: steht für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine Aryl-(C₁ bis C₄)-alkylgruppe,
- G²⁴: steht für eine Hydroxygruppe oder eine Gruppe -NG²⁶G²⁷, worin G²⁶ und G²⁷ unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe,
- G²⁵: Wasserstoffatom, ein Halogenatom oder eine (C₁ bis C₄)-Alkylgruppe,
mit der Maßgabe, dass G²⁴ in meta-Position oder ortho-Position zum Strukturfragment NG²³ der Formel bindet.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß verwendbaren Indolinderivate werden bevorzugt ausgewählt aus mindestens einer Verbindung der Formel (K6) und/oder aus mindestens einem physiologisch verträglichen Salz einer Verbindung gemäß Formel (K6), 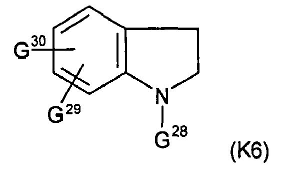 worin
- G²⁸: steht für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine Aryl-(C₁ bis C₄)-alkylgruppe,
- G²⁹: steht für eine Hydroxygruppe oder eine Gruppe -NG³¹G³², worin G³¹ und G³² unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe,
- G³⁰: Wasserstoffatom, ein Halogenatom oder eine (C₁ bis C₄)-Alkylgruppe,
mit der Maßgabe, dass G²⁹ in meta-Position oder ortho-Position zum Strukturfragment NG²⁸ der Formel bindet.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyf}amino)ethanol, 2-({3-[(2-Hydroxyethyl)amino]-2-methoxy-5-methylphenyl}amino)ethanol, 2-({3-[(2-Hydroxyethyl)amino]-4, 5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Im folgenden werden Beispiele für die als Substituenten der Verbindungen der Formeln (K1) bis (K6) genannten Reste aufgezählt: Beispiele für (C₁ bis C₄)-Alkylreste sind die Gruppen -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃.
Erfindungsgemäße Beispiele für (C₃ bis C₆)-Cycloalkylgruppen sind die Cyclopropyl, die Cyclopentyl und die Cyclohexylgruppe.
Erfindungsgemäße Beispiele für (C₁ bis C₄)-Alkoxyreste sind -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, -OC(CH₃)₃, insbesondere eine Methoxy- oder eine Ethoxygruppe.
Weiterhin können als bevorzugte Beispiele für eine (C₁ bis C₄)-Monohydroxyalkylgruppe -CH₂OH, -CH₂CH₂OH, -CH₂CH₂CH₂OH, -CH₂CH(OH)CH₃, -CH₂CH₂CH₂CH₂OH genannt werden, wobei die Gruppe -CH₂CH₂OH bevorzugt ist.
Ein besonders bevorzugtes Beispiel einer (C₂ bis C₄)-Polyhydroxyalkylgruppe ist die 1,2-Dihydroxyethylgruppe.
Beispiele für Halogenatome sind F-, Cl- oder Br-Atome, Cl-Atome sind ganz besonders bevorzugte Beispiele.
Beispiele für stickstoffhaltige Gruppen sind insbesondere -NH₂, (C₁ bis C₄)-Monoalkylaminogruppen, (C₁ bis C₄)-Dialkylaminogruppen, (C₁ bis C₄)-Trialkylammoniumgruppen, (C₁ bis C₄)-Monohydroxyalkylaminogruppen, Imidazolinium und -NH₃⁺.
Beispiele für (C₁ bis C₄)-Monoalkylaminogruppen sind -NHCH₃, -NHCH₂CH₃, -NHCH₂CH₂CH₃, -NHCH(CH₃)₂.
Beispiele für (C₁ bis C₄)-Dialkylaminogruppe sind -N(CH₃)₂, -N(CH₂CH₃)₂.
Beispiele für (C₁ bis C₄)-Alkoxy-(C₁ bis C₄)-alkylgruppen sind die Gruppen -CH₂CH₂-O-CH₃,
-CH₂CH₂CH₂-O-CH₃, -CH₂CH₂-O-CH₂CH₃, -CH₂CH₂CH₂-O-CH₂CH₃, -CH₂CH₂-O-CH(CH₃)₂, -CH₂CH₂CH₂-O-CH(CH₃)₂.
Beispiele für (C₁ bis C₄)-Alkoxy-(C₁ bis C₄)-alkoxygruppen sind die Gruppen -O-CH₂CH₂-O-CH₃, -O-CH₂CH₂CH₂-O-CH₃, -O-CH₂CH₂-O-CH₂CH₃, -O-CH₂CH₂CH₂-O-CH₂CH₃, -O-CH₂CH₂-O-CH(CH₃)₂, -O-CH₂CH₂CH₂-O-CH(CH₃)₂
Beispiele für Hydroxy-(C₁ bis C₄)-alkoxyreste sind -O-CH₂OH, -O-CH₂CH₂OH, -O-CH₂CH₂CH₂OH, -O-CH₂CH(OH)CH₃, -O-CH₂CH₂CH₂CH₂OH.
Beispiele für (C₁ bis C₄)-Aminoalkylreste sind -CH₂NH₂, -CH₂CH₂NH₂, -CH₂CH₂CH₂NH₂, -CH₂CH(NH₂)CH₃, -CH₂CH₂CH₂CH₂NH₂.
Ein Beispiel für Arylgruppen ist die Phenylgruppe, die auch substituiert sein kann.
Beispiele für Aryl-(C₁ bis C₄)-alkylgruppen sind die Benzylgruppe und die 2-Phenylethylgruppe.

Als Oxofarbstoffvorprodukte kommt bevorzugt eine Kombination aus
- mindestens einer Verbindung, die mindestens eine reaktive Carbonylgruppe enthält (Komponente (Oxo 1))
   mit mindestens einer Verbindung (Komponente Oxo2)
- Verbindungen, ausgewählt aus
   (Oxo2a) CH-aciden Verbindungen
      und/oder aus
   (Oxo2b) Verbindungen mit primärer oder sekundärer Aminogruppe oder Hydroxygruppe, ausgewählt aus mindestens einer Verbindung der Gruppe, die gebildet wird aus primären oder sekundären aromatischen Aminen, stickstoffhaltigen heterozyklischen Verbindungen und aromatischen Hydroxyverbindungen
zum Einsatz.

Reaktive Carbonylverbindungen als Komponente (Oxo1) besitzen im Sinne der Erfindung mindestens eine Carbonylgruppe als reaktive Gruppe, welche mit der Komponente (Oxo2) unter Ausbildung einer kovalenten Bindung reagiert. Bevorzugte reaktive Carbonylverbindungen sind ausgewählt aus Verbindungen die mindestens eine Formylgruppe und/oder mindestens eine Ketogruppe, insbesondere mindestens eine Formylgruppe, tragen. Ferner sind erfindungsgemäß auch solche Verbindungen als Komponente (Oxo1) verwendbar, in denen die reaktive Carbonylgruppe derart derivatisiert bzw. maskiert ist, dass die Reaktivität des Kohlenstoffatoms der derivatisierten Carbonylgruppe gegenüber der Komponente (Oxo2) stets vorhanden ist. Diese Derivate sind bevorzugt Additionsverbindungen
a) von Aminen und deren Derivate unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente (Oxo1) leitet sich in diesem Fall c) von einem Aldehyd ab)
an das Kohlenstoffatom der Carbonylgruppe der reaktiven Carbonylverbindung.

Bevorzugte reaktive Carbonylverbindungen der Komponente (Oxo1) werden ausgewählt aus der Gruppe, bestehend aus Benzaldehyd und seinen Derivaten, Naphthaldehyd und seinen Derivaten, Zimtaldehyd und seinen Derivaten, 2,3,6,7-Tetrahydro-1 H,SH-benzo[ij]chinolizin-9-carboxaldehyd, 2,3,6,7-Tetrahydro-8-hydroxy-1 H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd),
2-Indolaldehyd, 3-Indolaldehyd, 1-Methylindol-3-aldehyd, 2-Methylindol-3-aldehyd, 2-(1',3',3'-Trimethyl-2-indolinyliden)-acetaldehyd, 1-Methylpyrrol-2-aldehyd, 4-Pyridinaldehyd, 2-Pyridinaldehyd, 3-Pyridinaldehyd, Pyridoxal, Antipyrin-4-aldehyd, Furfural, 5-Nitrofurfural, 2-Thenoyltrifluor-aceton, Chromon-3-aldehyd, 3-(5'-Nitro-2'-furyl)-acrolein, 3-(2'-Furyl)-acrolein und Imidazol-2-aldehyd, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 5-(4-Dimethylamino-1-naphthyl)penta-3,5-dienal,
9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure,
4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-benzolsulfonat, -p-toluolsulfonat, -methansulfonat, -perchlorat, -sulfat, -chlorid, -bromid, -iodid, -tetrachlorozinkat, -methylsulfat-, - trifluormethansulfonat, -tetrafluoroborat, Isatin, 1-Methyl-isatin, 1-Allyl-isatin, 1-Hydroxymethylisatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Ganz besonders bevorzugt werden als reaktive Carbonylkomponente im Rahmen der Oxofärbung Benzaldehyd und/oder Zimtaldehyd und/oder Naphthaldehyd und/oder mindestens ein Derivat dieser vorgenannten Aldehyde, die insbesondere einen oder mehrere Hydroxy-, Alkoxy- oder Aminosubstituenten tragen, verwendet. Bevorzugt wird dabei wiederum die reaktive Carbonylverbindung der Komponente (Oxo1) ausgewählt aus mindestens einer Verbindung gemäß Formel (AC-1), 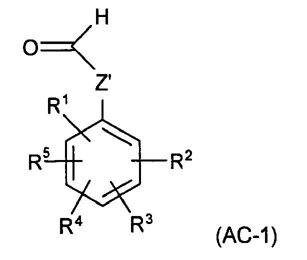 worin
- R¹ R² und R³ stehen unabhängig voneinander für ein Wasserstoffatom, ein Halogenatom, eine (C₁ bis C₆)-Alkylgruppe, eine (C₂ bis C₆)-Alkenylgruppe, eine Formylgruppe, eine Hydroxygruppe, eine C₁-C₆-Alkoxygruppe, eine (C₁ bis C₆)-Dialkylaminogruppe, eine Di(C₂-C₆-hydroxyalkyl)aminogruppe, eine Di(C₁ bis C₆)-alkoxy-(C₁ bis C₆)-alkyl)aminoguppe, eine (C₁ bis C₆)-Hydroxyalkyloxygruppe, eine Sulfonylgruppe, eine Carboxylgruppe, eine Sulfonsäuregruppe, eine Sulfonamidgruppe, eine Carbamoylgruppe, eine (C₂ bis C₆)-Acylgruppe, eine Acetylgruppe oder eine Nitrogruppe,
- Z' steht für eine direkte Bindung oder eine Vinylengruppe,
- R⁴ und R⁵ stehen für ein Wasserstoffatom oder bilden gemeinsam, zusammen mit dem Restmolekül einen 5- oder 6-gliederigen aromatischen oder aliphatischen Ring.

Die Derivate der Benzaldehyde, Naphthaldehyde bzw. Zimtaldehyde der reaktiven Carbonylverbindung gemäß Komponente (Oxo1) werden bevorzugt ausgewählt aus mindestens einer Verbindung der Gruppe, bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd, 4-Dimethylamino-2-methoxybenzaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxybenzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxybenzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-diiod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 3-Hydroxy-4-nitrobenzaldehyd, 2-Hydroxy-3-methoxy-5-nitrobenzaldehyd, 5-Nitrovanillin, 2,5-Dinitrosalicylaldehyd, 5-Brom-3-nitrosalicylaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 3-Carboxy-4-hydroxy-benzaldehyd, 5-Carboxyvanillin, 3-Carboxy-4-hydroxy-5-methylbenzaldehyd, 3-Carboxy-5-ethoxy-4-hydroxybenzaldehyd, 3-Carboxy-4-hydroxybenzaldehyd, 5-Carboxyvanillin, 3-Carboxy-4-hydroxy-5-methylbenzaldehyd, 3-Carboxy-5-ethoxy-4-hydroxybenzaldehyd, 3-Allyl-4-hydroxybenzaldehyd, 3-Allyl-4-hydroxy-5-methoxybenzaldehyd, 3-Allyl-4-hydroxy-5-methylbenzaldehyd, 3-Allyl-5-brom-4-hydroxybenzaldehyd, 3,5-Diallyl-4-hydroxybenzaldehyd, 3-Allyl-5-carboxy-4-hydroxybenzaldehyd (3-Allyl-5-formyl-2-hydroxybenzoesäure), 3-Allyl-4-hydroxy-5-formylbenzaldehyd (5-Allyl-4-hydroxyisophthalaldehyd) und Piperonal.

Als CH-acide Verbindungen werden im Allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Prinzipiell sind der Auswahl der CH-aciden Verbindungen keine Grenzen gesetzt, solange nach der Kondensation mit den reaktiven Carbonylverbindungen der Komponente (Oxo1) eine für das menschliche Auge sichtbar farbige Verbindung erhalten wird. Es handelt sich erfindungsgemäß bevorzugt um solche CH-aciden Verbindungen, welche einen aromatischen und/oder einen heterozyklischen Rest enthalten. Der heterozyklische Rest kann wiederum aliphatisch oder aromatisch sein. Besonders bevorzugt werden die CH-aciden Verbindungen ausgewählt aus heterozyklischen Verbindungen, insbesondere kationischen, heterozyklischen Verbindungen.

Ganz besonders bevorzugt wird als Komponente (Oxo2a) mindestens eine CH-acide Verbindung mit einem aromatischen oder aliphatischen, heterozyklischen Grundkörper eingesetzt, die ausgewählt wird aus zyklischen Oniumverbindungen mit der Struktureinheit der Formel (CH-1) und/oder Verbindungen der Formel (CH-2), 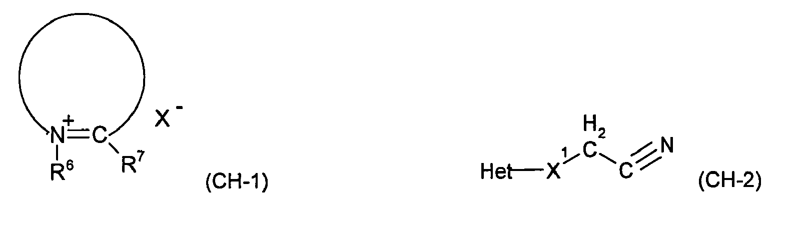 worin
- R⁶ steht für eine lineare oder cyclische (C₁ bis C₆)-Alkylgruppe, eine (C₂ bis C₆)-Alkenylgruppe, eine gegebenenfalls substituierte Arylgruppe, eine gegebenenfalls substituierte Heteroarylgruppe, eine Aryl-(C₁ bis C₆)-alkylgruppe, eine (C₁ bis C₆)-Hydroxyalkylgruppe, eine (C₂ bis C₆)-Polyhydroxyalkylgruppe, eine (C₁ bis C₆)-Alkoxy-(C₁ bis C₆)-alkylgruppe, eine Gruppe R¹R¹¹N-(CH₂)ₘ-, worin R^{I} und R^{II} stehen unabhängig voneinander für ein Wasserstoffatom, eine (C₁ bis C₄)-Alkylgruppe, eine (C₁ bis C₄)-Hydroxyalkylgruppe oder eine Aryl-(C₁ bis C₆)-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6,
- R⁷ steht für eine (C₁ bis C₆)-Alkylgruppe, insbesondere für eine Methylgruppe,
- X- steht für ein physiologisch verträgliches Anion,
- der Zyklus der Formel (CH-1) repräsentiert alle Ringstrukturen, die zusätzlich weitere Heteroatome wie Stickstoff, Sauerstoff oder Schwefel enthalten können und ferner ankondensierte Ringstrukturen tragen können, wobei alle diese Ringsstrukturen zusätzliche Substituenten tragen können,
- Het steht für einen gegebenenfalls substituierten Heteroaromaten,
- X¹ steht für eine direkte Bindung oder eine Carbonylgruppe.

Bevorzugte Ringstrukturen, die die Struktureinheit der Formel (CH-1) tragen, werden erfindungsgemäß bevorzugt ausgewählt aus 3H-Indolium, Benzothiazolium, Benzoxazolium, 1,2-Dihydro-2-oxopyrimidinium, Chinolinium, Chinoxalinium oder Pyridinium.

Erfindungsgemäß eignen sich Verbindungen gemäß Formel (CH-2) besonders gut solche, in denen sich der Rest Het gemäß Formel (CH-2) ableitet von einem der Heteroaromaten Furan, Thiophen, Pyrrol, Isoxazol, Isothiazol, Imidazol, Oxazol, Thiazol, Pyridin, Pyridazin, Pyrimidin, Pyrazin, 1,2,3-Triazin, 1,2,4-Triazin, 1,3,5-Triazin, Benzopyrrol, Benzofuran, Benzothiophen, Benzimidazol, Benzoxazol, Indazol, Benzoisoxazol, Benzoisothiazol, Indol, Chinolin, Isochinolin, Cinnolin, Phthalazin, Chinazolin, Chinoxalin, Acridin, Benzochinolin, Benzoisochinolin, Phenazin, Benzocinnolin, Benzochinazolin, Benzochinoxalin, Phenoxazin, Phenothiazin, Nephthyridin, Phenanthrolin, Indolizin, Chinolizin, Carbolin, Purin, Pteridin oder Cumarin, wobei die vorgenannten Heteroaromaten mit mindestens einer Gruppe ausgewählt aus einem Halogenatom, einer Nitrogruppe, einer Thiogruppe, einer Thio-(C₁ bis C₆)-alkylgruppe, einer Heteroarylgruppe, einer Arylgruppe, einer (C₁ bis C₆)-Alkylgruppe, einer (C₁ bis C₆)-Alkoxygruppe, einer Hydroxygruppe, einer (C₂ bis C₆)-Hydroxyalkylgruppe, einer (C₂ bis C₆)-Polyhydroxyalkylgruppe, einer (C₁ bis C₆)-Alkoxy-(C₁ bis C₆)-alkylgruppe, einer Aryl-(C₁ bis C₆)-alkylgruppe, einer Aminogruppe, einer (C₁ bis C₆)-Monoalkylaminogruppe, einer (C₁ bis C₆)-Dialkylaminogruppe, eine Dialkylaminoalkylgruppe -(CH₂)ₙ-NR'R", worin n eine ganze Zahl von 2 und 6 ist und R' und R" unabhängig voneinander eine lineare oder verzweigte Alkylgruppe bedeutet, welche gegebenenfalls zusammen einen Ring bilden können, substituiert sein können.

Vorzugsweise werden die Verbindungen gemäß Formel (CH-2) ausgewählt aus mindestens einer Verbindung der Gruppe bestehend aus 2-(2-Furoyl)-acetonitril, 2-(5-Brom-2-furoyl)-acetonitril, 2-(5-Methyl-2-trifluormethyl-3-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1*H*-Benzimidazol-2-ylacetonitril, 1*H*-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 8-Cyanacetyl-7-methoxy-4-methylcumarin, 2-(2-Isopropyl-5,6-benzochinolin-4-oyl)-acetonitril, 2-(2-Phenyl-5,6-benzochinolin-4-oyl)-acetonitril, 2-(Chinoxalin-2-yl)-acetonitril, 2-(Cumaron-2-yl)-acetonitril, 6,7-Dichlor-5-(cyanoacetyl)-2,3-dihydro-1-benzofuran-2-carbonsäure-tert.-butylester, 2-(6-Hydroxy-4,7-dimethoxy-1-benzofuran-5-oyl)-acetonitril und 2-(1-Phenyl-1,4-dihydrothiochromeno[4,3-c]pyrazol-3-oyl)-acetonitril.

Die CH-aciden Verbindungen der Oxofarbstoffvorprodukte der Komponente (Oxo2a) werden bevorzugt aus mindestens einer Verbindung der Formel (CH-3) ausgewählt, 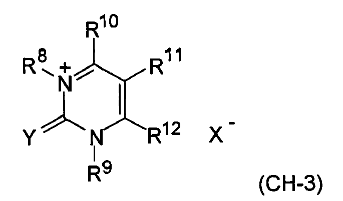 worin
- R⁸ und R⁹ stehen unabhängig voneinander für eine lineare oder cyclische (C₁ bis C₆)-Alkylgruppe, eine (C₂ bis C₆)-Alkenylgruppe, eine gegebenenfalls substituierte Arylgruppe, eine gegebenenfalls substituierte Heteroarylgruppe, eine Aryl-(C₁ bis C₆)-alkylgruppe, eine (C₁ bis C₆)-Hydroxyalkylgruppe, eine (C₂ bis C₆)-Polyhydroxyalkylgruppe, eine (C₁ bis C₆)-Alkoxy-(C₁ bis C₆)-alkylgruppe, eine Gruppe R^{I}R^{II}N-(CH₂)ₘ-, worin R^{I} und R^{II} stehen unabhängig voneinander für ein Wasserstoffatom, eine (C₁ bis C₄)-Alkylgruppe, eine (C₁ bis C₄)-Hydroxyalkylgruppe oder eine Aryl-(C₁ bis C₄)-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6,
- R¹⁰ und R¹² stehen unabhängig voneinander für ein Wasserstoffatom oder eine C₁-C₆-Alkylgruppe, wobei mindestens einer der Reste R¹⁰ und R¹² eine (C₁ bis C₆)-Alkylgruppe bedeutet,
- R¹¹ steht für ein Wasserstoffatom, eine (C₁ bis C₆)-Alkylgruppe, eine (C₁ bis C₆)-Hydroxyalkylgruppe, eine (C₂ bis C₆)-Polyhydroxyalkylgruppe, eine (C₁ bis C₆)-Alkoxygruppe, eine (C₁ bis C₆)-Hydroxyalkoxygruppe, eine Gruppe R^{III}R^{IV}N-(CH₂)_{q}-, worin R^{III} und R^{IV} stehen unabhängig voneinander für ein Wasserstoffatom, eine (C₁ bis C₆)-Alkylgruppe, eine (C₁ bis C₆)-Hydroxyalkylgruppe oder eine Aryl-(C₁ bis C₆)-alkylgruppe und q steht für eine Zahl 1, 2, 3, 4, 5 oder 6, wobei der Rest R¹¹ zusammen mit einem der Reste R¹⁰ oder R¹² einen 5- oder 6-gliedrigen aromatischen Ring bilden kann, der gegebenenfalls mit einem Halogenatom, einer (C₁ bis C₆)-Alkylgruppe, einer (C₁ bis C₆)-Hydroxyalkylgruppe, einer (C₂ bis C₆)-Polyhydroxyalkylgruppe, einer (C₁ bis C₆)-Alkoxygruppe, einer (C₁ bis C₆)-Hydroxyalkoxygruppe, einer Nitrogruppe, einer Hydroxygruppe, einer Gruppe R^{V}R^{VI}N-(CH₂)ₛ-, worin R^{v} und R^{VI} stehen unabhängig voneinander für ein Wasserstoffatom, eine (C₁ bis C₆)-Alkylgruppe, eine (C₁ bis C₆)-Hydroxyalkylgruppe oder eine Aryl-(C₁ bis C₆)-alkylgruppe und s steht für eine Zahl 0, 1, 2, 3, 4, 5 oder 6 substituiert sein kann,
- Y steht für ein Sauerstoffatom, ein Schwefelatom oder eine Gruppe NR^{VII}, worin R^{VII} steht für ein Wasserstoffatom, eine Arylgruppe, eine Heteroarylgruppe, eine (C₁ bis C₆)-Alkylgruppe oder eine Aryl-(C₁ bis C₆)-alkylgruppe,
- X- steht für ein physiologisch verträgliches Anion.

Mindestens eine Gruppe R¹⁰ oder R¹² gemäß Formel (CH-3) steht zwingend für eine (C₁ bis C₆)-Alkylgruppe. Diese Alkylgruppe trägt an deren α-Kohlenstoffatom bevorzugt mindestens zwei Wasserstoffatome. Besonders bevorzugte Alkylgruppen sind die Methyl-, Ethyl-, Propyl-, n-Butyl-, iso-Butyl, n-Pentyl-, neo-Pentyl-, n-Hexylgruppe. Ganz besonders bevorzugt stehen R¹⁰ und R¹² unabhängig voneinander für Wasserstoff oder eine Methylgruppe, wobei mindestens eine Gruppe R¹⁰ oder R¹² eine Methylgruppe bedeutet.

In einer bevorzugten Ausführungsform steht Y der Formel (CH-3) für ein Sauerstoff- oder ein Schwefelatom, besonders bevorzugt für ein Sauerstoffatom.

Der Rest R⁸ der Formel (CH-3) wird bevorzugt ausgewählt aus einer (C₁ bis C₆)-Alkylgruppe (besonders bevorzugt einer Methylgruppe), einer (C₂ bis C₆)-Alkenylgruppe (insbesondere einer Allylgruppe), einer (C₂ bis C₆)-Hydroxyalkylgruppe (insbesondere eine 2-Hydroxyethylgruppe) oder einer gegebenenfalls substituierten Benzylgruppe.

R¹¹ der Formel (CH-3) steht bevorzugt für ein Wasserstoffatom.

Besonders bevorzugt stehen in Formel (CH-3) die Reste R⁹, R¹⁰ und R¹² für eine Methylgruppe, der Rest R¹¹ für ein Wasserstoffatom, Y für ein Sauerstoff- oder ein Schwefelatom und der Rest R⁸ wird ausgewählt aus einer (C₁ bis C₆)-Alkylgruppe (besonders bevorzugt einer Methylgruppe), einer (C₂ bis C₆)-Alkenylgruppe (insbesondere einer Allylgruppe), einer (C₂ bis C₆)-Hydroxyalkylgruppe (insbesondere eine 2-Hydroxyethylgruppe) oder einer gegebenenfalls substituierten Benzylgruppe.

Vorzugsweise sind die Verbindungen gemäß Formel (CH-3) ausgewählt aus einer oder mehrerer Verbindungen der Gruppe von Salzen mit physiologisch verträglichem Gegenion X⁻, die gebildet wird aus Salzen des
1,2-Dihydro-1,3,4,6-tetramethyl-2-oxo-pyrimidiniums,
1,2-Dihydro-1,3-diethyl-4,6-dimethyl-2-oxo-pyrimidiniums,
1,2-Dihydro-1,3-dipropyl-4,6-dimethyl-2-oxo-pyrimidiniums,
1,2-Dihydro-1,3-di(2-hydroxyethyl)-4,6-dimethyl-2-oxo-pyrimidiniums,
1,2-Dihydro-1,3-diphenyl-4,6-dimethyl-2-oxo-pyrimidiniums,
1,2-Dihydro-1,3,4-trimethyl-2-oxo-pyrimidiniums,
1,2-Dihydro-1,3-diethyl-4-methyl-2-oxo-pyrimidiniums,
1,2-Dihydro-1,3-dipropyl-4-methyl-2-oxo-pyrimidiniums,
1,2-Dihydro-1,3-di(2-hydroxyethyl)-4-methyl-2-oxo-pyrimidiniums,
1,2-Dihydro-1,3-diphenyl-4-methyl-2-oxo-pyrimidiniums,
1-Allyl-1,2-dihydro-3,4,6-trimethyl-2-oxo-pyrimidiniums,
1,2-Dihydro-1-(2-hydroxyethyl)-3,4,6-trimethyl-2-oxo-pyrimidiniums,
1,2-Dihydro-1,3,4,6-tetramethyl-2-thioxo-pyrimidiniums,
1,2-Dihydro-1,3-diethyl-4,6-dimethyl-2-thioxo-pyrimidiniums,
1,2-Dihydro-1,3-dipropyl-4,6-dimethyl-2-thioxo-pyrimidiniums,
1,2-Dihydro-1,3-di(2-hydroxyethyl)-4,6-d imethyl-2-thioxo-pyrimidiniums,
1,2-Dihydro-1,3-diphenyl-4,6-dimethyl-2-thioxo-pyrimidiniums,
1,2-Dihydro-1,3,4-trimethyl-2-thioxo-pyrimidiniums,
1,2-Dihydro-1,3-diethyl-4-methyl-2-thioxo-pyrimidiniums,
1,2-Dihydro-1,3-dipropyl-4-methyl-2-thioxo-pyrimidiniums,
1,2-Dihydro-1,3-di(2-hydroxyethyl)-4-methyl-2-thioxo-pyrimidiniums,
1,2-Dihydro-1,3-diphenyl-4-methyl-2-thioxo-pyrimidiniums,
1,2-Dihydro-3,4-dimethyl-2-oxo-chinazoliniums und
1,2-Dihydro-3,4-dimethyl-2-thioxo-chinazoliniums.

Ganz besonders bevorzugte Verbindungen gemäß Formel (CH-3) werden ausgewählt aus einer oder mehrerer Verbindungen der Gruppe von Salzen mit physiologisch verträglichem Gegenion X-, die gebildet wird aus Salzen des
1,2-Dihydro-1,3,4,6-tetramethyl-2-oxo-pyrimidiniums,
1,2-Dihydro-1,3,4-trimethyl-2-oxo-pyrimidiniums,
1-Allyl-1,2-d ihydro-3,4,6-trimethyl-2-oxo-pyrimidiniums,
1,2-Dihydro-1-(2-hydroxyethyl)-3,4,6-trimethyl-2-oxo-pyrimidiniums und
1,2-Dihydro-1,3,4,6-tetramethyl-2-thioxo-pyrimidiniums.

X⁻ steht in den Formeln (CH-1) und (CH-3) sowie in obigen Listen bevorzugt für Halogenid, Benzolsulfonat, p-Toluolsulfonat, (C₁ bis C₄)-Alkansulfonat, Trifluormethansulfonat, Perchlorat, 0.5 Sulfat, Hydrogensulfat, Tetrafluoroborat, Hexafluorophosphat oder Tetrachlorozinkat. Besonders bevorzugt werden die Anionen Chlorid, Bromid, Iodid, Hydrogensulfat oder p-Toluolsulfonat als X-eingesetzt.

Verbindungen der Formel (CH-3) werden erfindungsgemäß besonders bevorzugt verwendet.

Die CH-aciden Verbindungen der Oxofarbstoffvorprodukte der Komponente (Oxo2a) werden ganz besonders bevorzugt ausgewählt aus mindestens einer Verbindung der Gruppe, bestehend aus 2-(2-Furoyl)-acetonitril, 2-(5-Brom-2-furoyl)-acetonitril, 2-(5-Methyl-2-trifluormethyl-3-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1 H-Benzimidazol-2-ylacetonitril, 1 H-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 8-Cyanacetyl-7-methoxy-4-methylcumarin, 2-(2-Isopropyl-5,6-benzochinolin-4-oyl)-acetonitril, 2-(2-Phenyl-5,6-benzochinolin-4-oyl)-acetonitril, 2-(Chinoxalin-2-yl)-acetonitril, 2-(Cumaron-2-yl)-acetonitril, 6,7-Dichlor-5-(cyanoacetyl)-2,3-dihydro-1-benzofuran-2-carbonsäure-tert.-butylester, 2-(6-Hydroxy-4,7-dimethoxy-1-benzofuran-5-oyl)-acetonitril, 2-(1-Phenyl-1,4-dihydrothiochromeno[4,3-c]pyrazol-3-oyl)-acetonitril, 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 2,3-Dimethyl-benzothiazoliumiodid, 2,3-Dimethyl-benzothiazolium-p-toluolsulfonat, 1,4-Dimethylchinolinium-iodid, 1,2-Dimethylchinolinium-iodid, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, 1,2,3-Trimethylchinoxalinium-p-toluolsulfonat, 1-Allyl-1,2-dihydro-3,4,6-trimethyl-2-oxopyrimidinium-chlorid, 1,2-Dihydro-1-(2-hydroxyethyl)-3,4,6-trimethyl-2-oxopyrimidinium-chlorid, 1,2-Dihydro-1,3,4,6-tetramethyl-2-oxo-pyrimidinium-chlorid, 1,2-Dihydro-1,3-diethyl-4,6-dimethyl-2-oxo-pyrimidinium-chlorid, 1,2-Dihydro-1,3-dipropyl-4,6-dimethyl-2-oxo-pyrimidinium-chlorid, 1-Allyl-1,2-dihydro-3,4,6-trimethyl-2-oxopyrimidinium-hydrogensulfat, 1,2-Dihydro-1-(2-hydroxyethyl)-3,4,6-trimethyl-2-oxopyrimidinium-hydrogensulfat, 1,2-Dihydro-1,3,4,6-tetramethyl-2-oxo-pyrimidinium-hydrogensulfat, 1,2-Dihydro-1,3-diethyl-4,6-dimethyt-2-oxo-pyrimidinium-hydrogensulfat, 1,2-Dihydro-1,3-dipropyl-4,6-dimethyl-2-oxo-pyrimidinium-hydrogensulfat, 1,2-Dihydro-1,3,4-trimethyl-2-oxo-pyrimidinium-chlorid, 1,2-Dihydro-1,3,4-trimethyl-2-oxo-pyrimidinium-hydrogensulfat, 1,2-Dihydro-1,3-diethyl-4-methyl-2-oxo-pyrimidinium-chlorid, 1,2-Dihydro-1,3-diethyl-4-methyl-2-oxo-pyrimidinium-hydrogensulfat, 1,2-Dihydro-1,3-dipropyl-4-methyl-2-oxo-pyrimidinium-chlorid, 1,2-Dihydro-1,3-dipropyl-4-methyl-2-oxo-pyrimidinium-hydrogensulfat, 1,2-Dihydro-1,3,4,6-tetramethyl-2-thioxo-pyrimidinium-chlorid, 1,2-Dihydro-1,3-diethyl-4,6-dimethyl-2-thioxo-pyrimidinium-chlorid, 1,2-Dihydro-1,3-dipropyl-4,6-dimethyl-2-thioxo-pyrimidinium-chlorid, 1,2-Dihydro-1,3,4,6-tetramethyl-2-thioxo-pyrimidinium-hydrogensulfat, 1,2-Dihydro-1,3-dipropyl-4,6-dimethyl-2-thioxo-pyrimidinium-hydrogensulfat, 1,2-Dihydro-1,3,4-trimethyl-2-thioxo-pyrimidinium-chlorid, 1,2-Dihydro-1,3,4-trimethyl-2-thioxo-pyrimidinium-hydrogensulfat, 1,2-Dihydro-1,3-diethyl-4-methyl-2-thioxo-pyrimidinium-chlorid, 1,2-Dihydro-1,3-diethyl-4-methyl-2-thioxo-pyrimidinium-hydrogensulfat, 1,2-Dihydro-1,3-dipropyl-4-methyl-2-thioxo-pyrimidinium-chlorid und 1,2-Dihydro-1,3-dipropyl-4-methyl-2-thioxo-pyrimidinium-hydrogensulfat.

Des Weiteren kann als Komponente (Oxo2b) mindestens ein Oxidationsfarbstoffvorprodukt mit mindestens einer primären oder sekundären Aminogruppe und/oder mindestens einer Hydroxygruppe verwendet werden. Bevorzugt geeignete Vertreter finden sich unter der Ausführung der Oxidationsfarbstoffvorprodukte. Es ist jedoch erfindungsgemäß bevorzugt, wenn die Verbindungen der Komponente (Oxo2) nur unter CH-aciden Verbindungen ausgewählt werden.

Die voranstehend genannten Verbindungen der Komponente (Oxo1) sowie der Komponente (Oxo2) werden, wenn sie zum Einsatz kommen, jeweils vorzugsweise in einer Menge von 0,03 bis 65 mmol, insbesondere von 1 bis 40 mmol, bezogen auf 100 g des gesamten Mittels, verwendet.

Bei den direktziehenden Farbstoffen handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden.

### Anionische direktziehende Farbstoffe: ,

Als anionische direktziehende Farbstoffe eignen sich insbesondere 6-Hydroxy-5-[(4-sulfophenyl)azo]-2-naphthalinsulfonsäuredinatriumsalz (C.I. 15,985; Food Yellow No. 3; FD&C Yellow No. 6), 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 4-((4-Amino-3-sulfophenyl)azo)benzolsulfonsäure-dinatriumsalz (C.I. 13,015, Acid Yellow 9), 5-Hydroxy-1-(4-sulfophenyl)-4-[(4-sulfophenyl)azo]pyrazol-3-carbonsäure-trinatriumsalz (C.I. 19,140; Food Yellow No. 4; Acid Yellow 23), 3-[(4-Phenylamino)phenyl]azobezolsulfonsäuresäure-natriumsalz (C.I. 13,065; Ki406; Acid Yellow 36), 9-(2-Carboxyphenyl)-6-hydroxy-3H-xanthen-3-on (C.I. 45,350; Acid Yellow 73; D&C Yellow No. 8), 5-[(2,4-Dinitrophenyl)amino]-2-phenylaminobenzolsulfonsäure-natriumsalz (C.I. 10,385; Acid Orange 3), 4-[(2,4- Dihydroxyphenyl)azo]-benzolsulfonsäure-natriumsalz (C.I. 14,270; Acid Orange 6), 4-[(2-Hydroxynaphth-1-yl)azo]-benzolsulfonsäure-natriumsalz (C.1. 15,510; Acid Orange 7), 4-[(2,4-Dihydroxy-3-[(2,4-dimethylphenyl)azo]-phenyl)azo]-benzolsulfonsäure-natriumsalz (C.I. 20,170; Acid Orange 24), 4-Hydroxy-3-[(2-methoxyphenyl)azo]-1-naphthalinsulfonsäure-natriumsalz (C.I. 14,710; Acid Red 4), 4-Hydroxy-3-[(4-sulfonaphth-1-yl)azo]-1-naphthalin-sulfonsäure-dinatriumsalz (C.I. 14,720; Acid Red No.14), 6- Hydroxy-5-[(4-sulfonaphth-1-yl)azo]-2,4-naphthalin-disulfonsäure-trinatriumsalz (C.I. 16,255; Ponceau 4R; Acid Red 18), 3-Hydroxy-4-[(4-sulfonaphth-1-yl)azo]-2,7-naphthalin-disulfonsäure-trinatriumsalz (C.I. 16,185; Acid Red 27), 8-Amino-1-hydroxy-2-(phenylazo)-3,6-naphthalin-disulfonsäure-dinatriumsalz (C.I. 17,200; Acid Red 33; Red 33), 5- (Acetylamino)-4-hydroxy-3-[(2-methylphenyl)azo]-2,7-naphthalin-disulfonsäure-dinatriumsalz (C.I. 18,065; Acid Red 35), 2-(3-Hydroxy-2,4,5,7-tetraiod-dibenzopyran-6-on-9-yl)-benzoesäure-dinatriumsalz (C.I. 45,430; Acid Red 51), N-[6-(Diethylamino)-9-(2,4-disulfophenyl)-3H-xanthen-3-yliden]-N-ethylethanammonium-hydroxid, inneres Salz, Natriumsalz (C.I. 45,100; Acid Red 52), 8-[(4-(Phenylazo)phenyl)azo]-7-naphthol-1,3-disulfonsäure-dinatriumsalz (C.I. 27,290; Acid Red 73), 2',4',5',7'-Tetrabrom-3',6'-dihydroxyspiro[isobenzofuran-1 (3H),9'-[9H]xanthen]-3-on-dinatriumsalz (C.I. 45,380; Acid Red 87), 2',4',5',7'-Tetrabrom-4,5,6,7-tetrachlor-3',6'- dihydroxyspiro[isobenzofuran-1(3H),9'[9H]xanthen]-3-on-dinatriumsalz (C.I. 45,410; Acid Red 92), 3',6'-Dihydroxy-4',5'-diiodospiro[isobenzofuran-1 (3H),9'(9H)- xanthen]-3-on-dinatriumsalz (C.I. 45425; Acid Red 95), 2-Hydroxy-3-((2- hydroxynaphth-1-yl)azo)-5-nitrobenzolsulfonsäure-natriumsalz (C.I. 15,685; Acid Red 184), 3-Hydroxy-4-(3-methyl-5-oxo-1-phenyl-4,5-dihydro-1 H-pyrazol-4-ylazo)-naphthalin-1-sulfonsäure-natriumsalz, Chrom-Komplex (Acid Red 195), 3-Hydroxy-4-[(4-methyl-2-sulfonphenyl)azo]-2-naphthalincarbonsäure-calciumsalz (C.I. 15,850:1; Pigment Red 57:1), 3-[(2,4-Dimethyl-5-sulfophenyl)azo]-4-hydroxy-1-naphthalin-sulfonsäure-dinatriumsalz (C.I. 14,700; Food Red No. 1; Ponceau SX; FD&C Red No. 4), 1,4- Bis[(2-sulfo-4-methylphenyl)amino]-9,10-anthrachinon-dinatriumsalz (C.I. 61,570; Acid Green 25), Bis[4-(dimethylamino)phenyl]-(3,7-disulfo-2-hydroxynaphth-1-yl)carbenium-inneres Salz, Natriumsalz (C.I. 44,090; Food Green No. 4; Acid Green 50), Bis[4-(diethylamino)-phenyl](2,4-disulfophenyl)carbenium-inneres Salz, Natriumsalz (2:1) (C.I. 42,045; Food Blue No. 3; Acid Blue 1), Bis[4-(diethylamino)phenyl](5-hydroxy-2,4-disulfophenyl)-carbenium-inneres Salz, Calciumsalz (2:1) (C.I. 42,051; Acid Blue 3), N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080; Acid Blue 7), (2-Sulfophenyl)di[4-(ethyl((4-sulfophenyl)methyl)amino)phenyl]-carbenium-dinatriumsalz Betain (C.I. 42,090; Acid Blue 9; FD&C Blue No. 1), 1-Amino-4-(phenylamino)-9,10-anthrachinon-2-sulfonsäure (C.I. 62,055; Acid Blue 25), 1-Amino-4-(cyclohexylamino)-9,10-anthrachinon-2-sulfonsäure-natriumsalz (C.I. 62045; Acid Blue 62), 2-(1,3-Dihydro-3-oxo-5-sulfo-2H-indol-2-yliden)-2,3-dihydro-3-oxo-1H-indol-5- sulfonsäure-dinatriumsalz (C.I. 73,015; Acid Blue 74), 9-(2-Carboxyphenyl)-3-[(2-methylphenyl)amino]-6-[(2-methyl-4-sulfophenyl)amino]xanthylium-inneres Salz, Natriumsalz (C.I. 45,190; Acid Violet 9), 1-Hydroxy-4-[(4-methyl-2- sulfophenyl)amino]-9,10-anthrachinon-natriumsalz (C.1. 60,730; D&C Violett No. 2; Acid Violet 43), Bis[3-nitro-4-[(4-phenylamino)-3-sulfo-phenylamino]-phenyl]-sulfon (C.I. 10,410; Acid Brown 13), 5-Amino-4-hydroxy-6-[(4-nitrophenyl)-azo]-3-(phenylazo)-2,7-naphthalin-disulfonsäure-dinatriumsalz (C.I. 20,470; Acid Black 1), 3-Hydroxy-4-[(2-hydroxynaphth-1-yl)azo]-7-nitro-1-naphthalin-sulfonsäure-chromkomplex (3:2) (C.I. 15,711; Acid Black 52), 4-(Acetylamino)-5-hydroxy-6-[(7-sulfo-4-[(4-sulfophenyl)azo]naphth-1-yl)azo]-1,7-naphthalindisulfonsäure-tetranatriumsalz (C.I. 28,440; Food Black No. 1), 3',3",5',5"-Tetrabromphenolsulfonphthalein (Bromphenolblau).

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

### Kationische direktziehende Farbstoffe:

Als kationische direktziehende Farbstoffe eignen sich insbesondere 9-(Dimethylamino)-benzo[a]phenoxazin-7-ium-chlorid (C.I. 51,175; Basic Blue 6), Di[4-(diethylamino)phenyl][4-(ethylamino)naphthyl]carbenium-chlorid (C.I. 42,595; Basic Blue 7), Di-(4-(dimethylamino)phenyl)-(4-(methyl-phenylamino)-naphthalin-1-yl)carbenium-chlorid (C.I. 42,563; Basic Blue 8), 3,7-Di(dimethylamino)phenothiazin-5-ium-chlorid (C.I. 52,015 Basic Blue 9), Di[4-(dimethylamino)phenyl][4-(phenylamino)naphthyl] carbenium-chlorid (C.I. 44,045; Basic Blue 26), 2-[(4-(Ethyl(2-hydroxyethyl)amino)phenyl)azo]-6-methoxy-3-methyl-benzothiazolium-methylsulfat (C.I. 11,154; Basic Blue 41), 8-Amino-2-brom-5-hydroxy-4-imino-6-[(3-(trimethylammonio)phenyl)amino]-1 (4H)-naphthalinon-chlorid (C.I. 56,059; Basic Blue No. 99), Bis[4- (dimethylamino)phenyl]-[4-(methylamino)phenyl]carbenium-chlorid (C.I. 42,535; Basic Violet 1), Tri(4-amino-3-methylphenyl)carbenium-chlorid (C.I. 42,520; Basic Violet 2), Tri[4-(dimethylamino)-phenyl]carbenium-chlorid (C.I. 42,555; Basic Violet 3), 2-[3,6-(Diethylamino)dibenzopyranium-9-yl]- benzoesäurechlorid (C.I. 45,170; Basic Violet 10), Di(4-aminophenyl)(4-amino-3- methylphenyl)carbeniumchlorid (C.I. 42,510 Basic Violet 14), 1,3-Bis[(2,4-diamino-5-methylphenyl)azo]-3-methylbenzol (C.I. 21,010; Basic Brown 4), 1-[(4-Aminophenyl)azo]-7-(trimethylammonio)- 2-naphthol-chlorid (C.I. 12,250; Basic Brown 16), 1-[(4-Amino-2-nitrophenyl)azo]-7-(trimethylammonio)-2-naphtholchlorid, 1-[(4-Amino-3-nitrophenyl)azo]-7-(trimethylammonio)-2-naphthol-chlorid (C.I. 12,251; Basic Brown 17), 3-[(4-Amino-2,5- dimethoxyphenyl)azo]-N,N,N-trimethylbenzolaminiumchlorid (C.I. 12,605, Basic Orange 69), 3,7-Diamino-2,8-dimethyl- 5-phenylphenazinium-chlorid (C.I. 50,240; Basic Red 2), 1,4-Dimethyl-5-[(4-(dimethylamino)phenyl)azo]-1,2,4-triazolium-chlorid (C.I. 11,055; Basic Red 22), 2-Hydroxy-1-[(2-methoxyphenyl)azo]-7-(trimethylammonio)-naphthalin-chlorid (C.I. 12,245; Basic Red 76), Di[4-(dimethylamino)phenyl]iminomethan-hydrochlorid (C.I. 41,000; Basic Yellow 2), 2-[2-((2,4- Dimethoxyphenyl)amino)ethenyl]-1,3,3-trimethyl-3H-indol-1-ium-chlorid (C.I. 48,055; Basic Yellow 11), 3-Methyl-1-phenyl-4-[(3-(trimethylammonio)phenyl)azo]-pyrazol-5-on-chlorid (C.I. 12,719; Basic Yellow 57), Bis[4-(diethylamino)phenyl]phenylcarbenium-hydrogensulfat (1:1) (C.I. 42,040; Basic Green 1), Di(4- (dimethylamino)phenyl)-phenylmethanol (C.1. 42,000; Basic Green 4), 1-(2- Morpholiniumpropylamino)-4-hydroxy-9,10-anthrachinon-methylsulfat, 1-[(3-(Dimethyl-propylaminium)-propyl)amino]-4-(methylamino)-9,10-anthrachinon-chlorid und direktziehende Farbstoffe, die einen Heterocyclus enthalten, der mindestens ein quaternäres Stickstoffatom aufweist.

Bevorzugte kationische direktziehenden Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

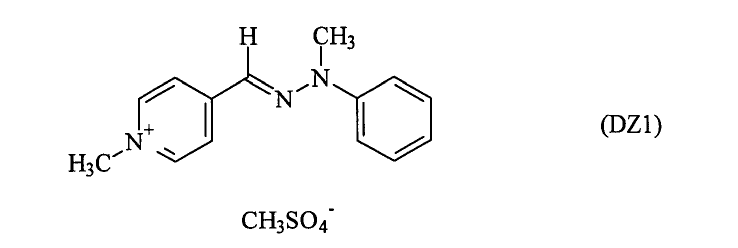

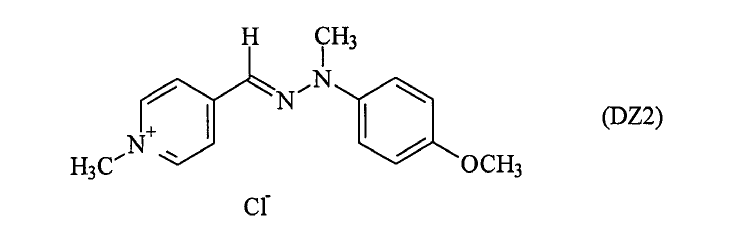

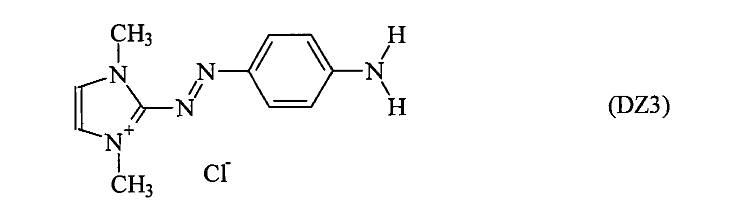

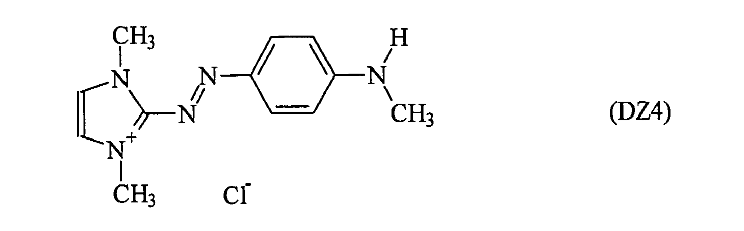

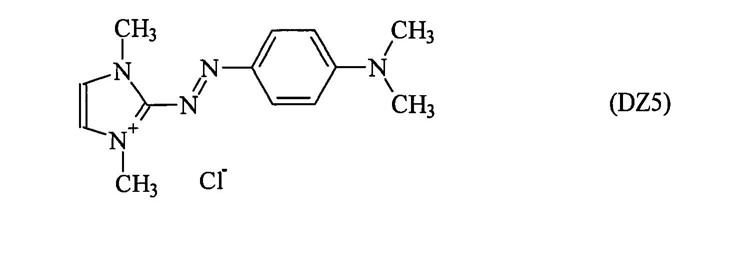

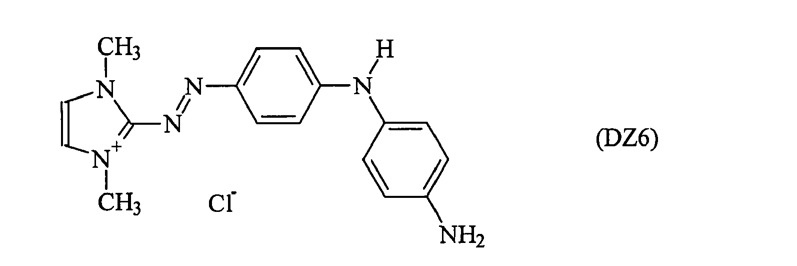

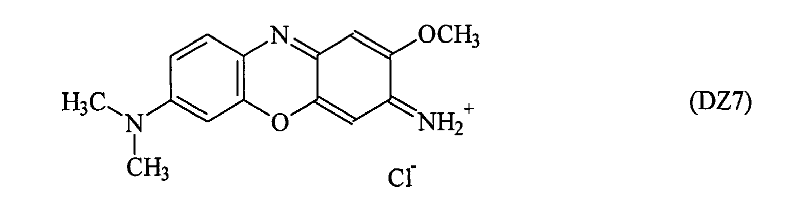

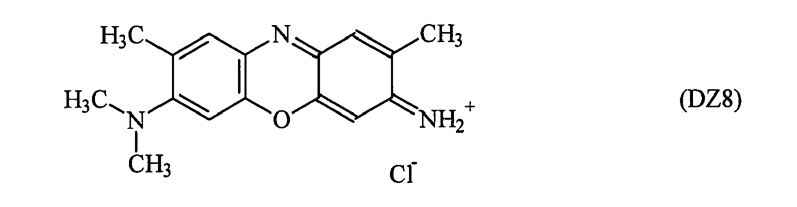

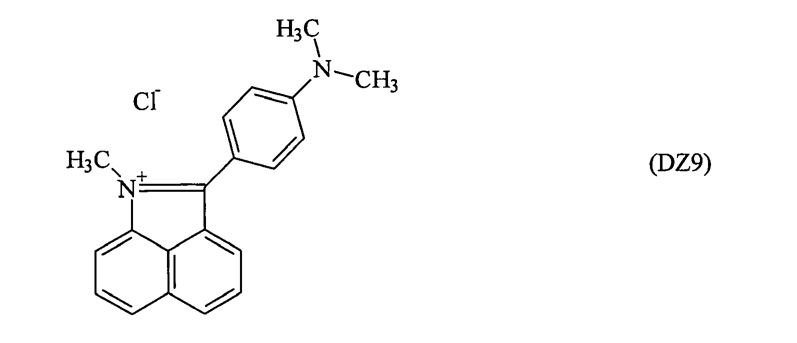

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

### Nichtionische direktziehende Farbstoffe:

Als nichtionische direktziehende Farbstoffe eignen sich insbesondere nichtionische Nitro- und Chinonfarbstoffe und neutrale Azofarbstoffe.

Geeignete blaue Nitrofarbstoffe sind insbesondere:
1,4-Bis[(2-hyd roxyethyl)amino]-2-nitrobenzol, 1-(2-Hydroxyethyl)amino-2-nitro-4-[di(2-hydroxyethyl)amino]-benzol (HC Blue 2), 1-Methylamino-4- [methyl-(2,3-dihydroxypropyl)amino]-2-nitrobenzol (HC Blue 6),1-[(2,3- Dihydroxypropyl)-amino]-4-[ethyl-(2-hydroxyethyl)amino]-2-nitrobenzol-hydrochlorid (HC Blue 9), 1-[(2,3-Dihydroxypropyl)amino]-4-[methyl-(2-hydroxyethyl)amino]-2-nitrobenzol (HC Blue 10), 4-[Di(2-hydroxyethyl)amino]-1-[(2-methoxyethyl)amino]-2-nitrobenzol (HC Blue 11), 4-[Ethyl-(2-hydroxyethyl)-amino]-1-[(2-hydroxyethyl)amino]-2-nitrobenzol-hydrochlorid (HC Blue 12), 2-((4-Amino-2-nitrophenyl)amino)-5-dimethylamino-benzoesäure (HC Blue 13), 1-Amino-3-methyl-4-[(2-hydroxyethyl)amino]-6-nitrobenzol (HC Violet 1), 1-(3-Hydroxypropylamino)-4-[di(2-hydroxyethyl)amino]-2-nitrobenzol (HC Violet 2), 1-(2-Aminoethylamino)-4-[di(2-hydroxyethyl)amino]-2-nitrobenzol, 4-(Di(2-hydroxyethyl)amino)-2-nitro-1-phenylamino-benzol.

Geeignete rote Nitrofarbstoffe sind insbesondere:
1-Amino-4-[(2-hydroxyethyl)amino]-2-nitrobenzol (HC Red 7), 2-Amino-4,6-dinitrophenol (Pikraminsäure) und deren Salze, 1,4-Diamino-2-nitrobenzol (C.I. 76,070), 4-Amino-2-nitro-diphenylamin (HC Red 1), 1-Amino-4-[di(2-hydroxyethyl)amino]-2-nitrobenzol-hydrochlorid (HC Red 13), 1-Amino-4-[(2-hydroxyethyl)-amino]-5-chlor-2-nitrobenzol, 4-Amino-1-[(2-hydroxyethyl)amino]-2-nitrobenzol (HC Red 3), 4-[(2-Hydroxyethyl)methylamino]-1-(methylamino)-2-nitrobenzol, 1-Amino-4-[(2,3-dihydroxypropyl)amino]-5-methyl-2-nitrobenzol, 1-Amino-4-(methylamino)-2-nitrobenzol, 4-Amino-2-nitro-1-[(prop-2-en-1-yl)-amino]-benzol, 4-Amino-3-nitrophenol, 4-[(2-Hydroxyethyl)-amino]-3-nitrophenol, 4-[(2-Nitrophenyl)amino]phenol (HC Orange 1), 1-[(2-Aminoethyl)amino]-4-(2-hydroxyethoxy)-2-nitrobenzol (HC Orange 2), 4-(2,3-Dihydroxypropoxy)-1-[(2-hydroxyethyl)amino]-2-nitrobenzol (HC Orange 3), 1-Amino-5-chlor-4-[(2,3-dihydroxypropyl)amino]-2-nitrobenzol (HC Red 10), 5-Chlor-1,4-[di(2,3-dihydroxypropyl)amino]-2-nitrobenzol (HC Red 11), 2-[(2- Hydroxyethyl)amino]-4,6-dinitrophenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol (HC Red BN), 2,5-Diamino-6-nitropyridin, 6-Amino-3-[(2-hydroxyethyl)amino]-2-nitropyridin, 3-Amino-6-[(2-hydroxyethyl)amino]-2-nitropyridin, 3-Amino-6-(ethylamino)-2-nitropyridin, 3-[(2-Hydroxyethyl)amino]-6-(methylamino)-2-nitropyridin, 3- Amino-6-(methylamino)-2-nitropyridin, 6-(Ethylamino)-3-[(2-hydroxyethyl)amino]-2-nitropyridin, 1,2,3,4-Tetrahydro-6-nitrochinoxalin, 7-Amino-3,4-dihydro-6-nitro-2H-1,4-benzoxazin (HC Red 14).

Geeignete gelbe Nitrofarbstoffe sind insbesondere:
1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxyethyl)amino]-5-nitrobenzol (HC Yellow 5), 4-[(2,3-Dihydroxypropyl)-amino]-3-nitro-1-trifluormethyl-benzol (HC Yellow 6), 2-[Di(2-hydroxyethyl)amino]-5-nitrophenol, 2-[(2-Hydroxyethyl)amino]-1-methoxy-5-nitrobenzol, 2-Amino-3-nitrophenol, 2-Amino-4-nitrophenol, 1-Amino-2-methyl-6-nitrobenzol, 1-(2-Hydroxyethoxy)-3-methylamino-4-nitrobenzol, 2,3-(Dihydroxy-propoxy)-3-methylamino-4-nitrobenzol, 3-[(2-Aminoethyl)amino]-1-methoxy-4-nitrobenzol-hydrochlorid (HC Yellow 9), 1-Chlor-2,4-bis[(2-hydroxyethyl)amino]-5-nitrobenzol (HC Yellow 10), 2-[(2-Hydroxyethyl)amino]-5-nitrophenol (HC Yellow 11), 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)amino]-3-nitro-1-trifluormethyl-benzol (HC Yellow 13), 4-[(2-Hydroxyethyl)-amino]-3-nitro-benzonitril (HC Yellow 14), 4-[(2-Hydroxyethyl)amino]-3-nitro-benzamid (HC Yellow 15) 3-[(2-Hydroxyethyl)amino]-4-methyl-1-nitrobenzol, 4-Chlor-3-[(2-hydroxyethyl)amino]-1-nitrobenzol.

Geeignete Chinonfarbstoffe sind insbesondere:
1,4-Di[(2,3-dihydroxypropyl)amino]-9,10-anthrachinon, 1,4-Di[(2-hydroxyethyl)amino]-9,10-anthrachinon (C.I. 61,545, Disperse Blue 23), 1-[(2-Hydroxyethyl)amino]-4-methylamino-9,10-anthrachinon (C.I. 61,505, Disperse Blue 3), 2-[(2-Aminoethyl)amino]-9,10-anthrachinon (HC Orange 5), 1-Amino-4-hydroxy-9,10-anthrachinon (C.I. 60,710, Disperse Red 15), 1-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbonsäure (C.I. 75,470, Natural Red 4), 1-[(3-Aminopropyl)amino]-4-methylamino-9,10-anthrachinon (HC Blue 8), 1-[(3-Aminopropyl)-amino]-9,10- anthrachinon (HC Red 8), 1,4-Diamino-2-methoxy-9,10-anthrachinon (C.I. 62,015, Disperse Red 11, Solvent Violet No. 26), 1,4-Dihydroxy-5,8-bis[(2-hydroxyethyl)amino]-9,10-anthrachinon (C.I. 62,500, Disperse Blue 7, Solvent Blue No. 69), 1,4-Diamino-9,10-anthrachinon (C.I. 61,100, Disperse Violet 1), 1-Amino-4-(methylamino)-9,10-anthrachinon (C.I. 61,105, Disperse Violet 4, Solvent Violet No. 12), 2-Hydroxy-3-methoxy-1,4-naphthochinon, 2,5-Dihydroxy-1,4-naphthochinon, 2-Hydroxy-3-methyl-1,4-naphthochinon, N-{6-[(3-Chlor-4-(methylamino)phenyl)imino]-4-methyl-3-oxo-1,4-cyclohexadien-1-yl}harnstoff (HC Red 9), 2-{{4-[Di(2-hydroxyethyl)amino]phenyl}amino}-5-[(2-hydroxyethyl)amino]-2,5-cyclohexadien-1,4-dion (HC Green 1), 5-Hydroxy-1,4-naphthochinon (C.I. 75,500, Natural Brown 7), 2-Hydroxy-1,4-naphthochinon (C.I. 75,480, Natural Orange 6), 1,2-Dihydro-2-(1,3-dihydro-3-oxo-2H-indol-2-yliden)-3H-indol-3-on (C.I. 73,000), 4-{{5-[(2-Hydroxyethyl) amino]-1-methyl-1 H- pyrazol-4-yl}imino}-4,5-dihydro-5-[(2-hydroxyethyl)-imino]-1-methyl-1H-Pyrazol-sulfat(1:1), Hydrat(1:1).

Geeignete neutrale Azofarbstoffe sind insbesondere:
1-[Di(2-hydroxyethyl)amino]-3-methyl-4-[(4-nitrophenyl)azo]-benzol (C.I. 11,210, Disperse Red 17), 1-[Di(2-hydroxyethyl)amino]-4-[(4-nitrophenyl)azo]-benzol (Disperse Black 9), 4-[(4-Aminophenyl)azo]-1-[di(2-hydroxyethyl)amino]-3-methylbenzol (HC Yellow 7), 2,6-Diamino-3-[(pyridin-3-yl)azo]-pyridin, 2-{[4-(Acetylamino)phenyl]azo}-4-methylphenol (C.I. 11855; Disperse Yellow 3), 4-[(4-Nitrophenyl)azo]-anilin (C.I. 11,005; Disperse Orange 3).

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, I, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders gut als Vorstufen naturanaloger Haarfarbstoffe geeignet sind Derivate des 5,6-Dihydroxyindols der Formel (RN1), 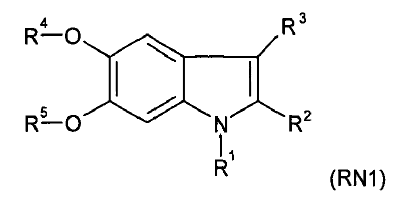 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindolins der Formel (RN2), 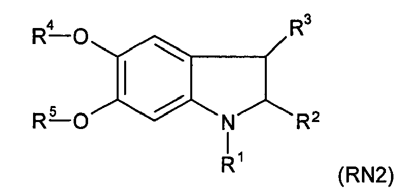 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Wenn das erfindungsgemäße Mittel Oxidationsfarbstoffvorprodukte oder insbesondere Vorstufen eines naturanalogen Farbstoffs auf Indol- bzw. Indolin-Basis, enthält, kann die eigentliche oxidative Färbung der Fasern grundsätzlich mit Luftsauerstoff erfolgen. Bevorzugt wird jedoch, bevorzugt im Falle der Oxidationsfarbstoffvorprodukte, ein zusätzliches Oxidationsmittel (d.h. ein von Luft verschiedenes Oxidationsmittel) eingesetzt. Wenn neben der Färbung mit den farbverändernden Komponenten ein Aufhelleffekt an menschlichem Haar gewünscht ist wird generell ein zusätzliches Oxidationsmittel verwendet. Als zusätzliches Oxidationsmittel kommen Persulfate, Chlorite und insbesondere Wasserstoffperoxid oder dessen Anlagerungsprodukte an Harnstoff, Melamin sowie an Natriumborat in Frage.

Die erfindungsgemäßen Mittel enthalten mindestens ein zusätzliches Oxidationsmittel, bevorzugt in einer Menge von 0,5 bis 12,0 Gew.%, insbesondere 6 bis 12 Gew.-%, jeweils bezogen auf das Gewicht des anwendungsbereiten Mittels.

Optional kann das erfindungsgemäße Mittel generell auch zusammen mit einem Oxidationsaktivator auf das Haar aufgebracht werden, der die Oxidation der Oxidationsfarbstoffvorprodukte durch das Oxidationsmittel, aktiviert. Als Oxidationsmittel dient, wie zuvor erwähnt, Luftsauerstoff oder zusätzliche Oxidationsmittel. Die Oxidationsaktivatoren werden bevorzugt ausgewählt aus der Gruppe, die gebildet wird aus Carbonaten, Hydrogencarbonaten, Carbamaten, Carbonsäureestern oder deren Salze, Aldehyden, insbesondere aliphatischen Aldehyden, 1,3-Dihydroxyaceton, Imidazol und seinen Derivaten, Alkali- und Ammoniumperoxidisulfaten, Metallionen, Iodiden, Chinonen und Enzymen.

Die Oxidationsaktivatoren sind bevorzugt in Mengen von 0.01 bis 5 Gew.%, bezogen auf das Gewicht des anwendungsbereiten Mittels, in den erfindungsgemäßen Mitteln enthalten.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und AI³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche, ein zusätzliches Oxidationsmittel enthaltende Haarfärbemittel wird zweckmäßigerweise unmittelbar vor der Anwendung durch Mischung einer Oxidationsmittelzusammensetzung mit einer Zusammensetzung, enthaltend die farbverändernden Komponenten, bevorzugt im Gewichtsverhältnisbereich von 1 zu 4 bis 4 zu 1, insbesondere von 1 zu 2 bis 2 zu 1, hergestellt, wobei die resultierende Mischung enthält
(i) eine wässrige Phase,
(ii) mindestens einen Fettstoff als Fettphase,
(iii) mindestens einen organischen UV-Absorber,
(iv) mindestens eine farbverändernde Komponente und
(v) mindestens ein Oxidationsmittel
mit der Maßgabe, dass sich in der resultierenden Mischung mindestens ein organischer UV-Absorber in mindestens einem Fettstoff der Fettphase befindet und die Entwickler-Komponenten sich in der wässrigen Phase befinden. Die Komponenten (i), (ii) und (iii) können vor dem Mischen sowohl in der Oxidationsmittelzusammensetzung, als auch in der Zusammensetzung, enthaltend die farbverändernden Komponenten enthalten sein, wobei letzteres erfindungsgemäß bevorzugt ist.

Eine weitere Ausführungsform des erfindungsgemäßen Mittels ist daher ein Färbemittel für keratinhaltige Fasern, insbesondere menschliches Haar, enthaltend
(i) eine wässrige Phase,
(ii) mindestens einen Fettstoff als Fettphase,
(iii) mindestens einen organischen UV-Absorber,
(iv) mindestens eine Entwicklerkomponente und gegebenenfalls mindestens eine Kupplerkomponente und
(v) gegebenenfalls mindestens ein Oxidationsmittel mit der Maßgabe, dass sich mindestens ein organischer UV-Absorber in mindestens einem Fettstoff der Fettphase befindet und die Entwickler-Komponenten und die gegebenenfalls enthaltenen Kupplerkomponenten sich in der wässrigen Phase befinden.

Zur Verstärkung des erfindungsgemäßen Effekts enthalten die erfindungsgemäßen Mittel aller Ausführungsformen bevorzugt zusätzlich mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- anionische Alkyloligoglykoside bzw. anionische Alkenyloligoglykosid-Derivate, ausgewählt aus Alkyl- und/oder Alkenyl-Oligoglykosidcarboxylaten, -sulfaten, - phosphaten und/oder -isethionaten, die sich von Alkyl- und/oder Alkenyloligoglykosiden der allgemeinen Formel (IV) ableiten,

   R-O-(G)ₚ (IV)

   mit der Bedeutung
   R C₆₋₂₂-Alkyl oder C₆₋₂₂-Alkenyl,
   G Glykosideinheit, die sich von einem Zucker mit 5 oder 6 Kohlenstoffatomen ableitet,
   p Zahl von 1 bis 10,
   insbesondere das Laurylglucosidcarboxylat, wie es als Plantapon^{®} LGC von Cognis Deutschland erhältlich ist,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H₁, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Weiterhin im Sinne der Erfindung nutzbare nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im Wesentlichen aus C₈- und C₁₀-Alkylgruppen,
- im Wesentlichen aus C₁₂- und C₁₄-Alkylgruppen,
- im Wesentlichen aus C₈- bis C₁₆-Alkylgruppen oder
- im Wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als nichtionische Tenside oder Emulgatoren dienen ebenso hydrophile Silikone. Diese werden beispielsweise aus den Verbindungen der Formeln (Si-6) und/oder (Si-7) ausgewählt. Insbesondere bevorzugte nichtionische Tenside auf Silikonbasis sind ausgewählt aus der Gruppe der Dimethiconcopolyole die bevorzugt alkoxyliert, insbesondere polyethoxyliert oder polypropoxyliert sind.

Unter Dimethiconcopolyolen werden erfindungsgemäß bevorzugt Polyoxyalkylen-modifizierte Dimethylpolysiloxane der allgemeinen Formeln (Si-6) oder (Si-7) verstanden: 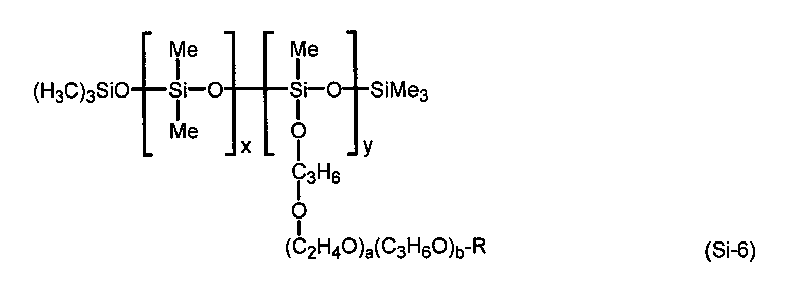 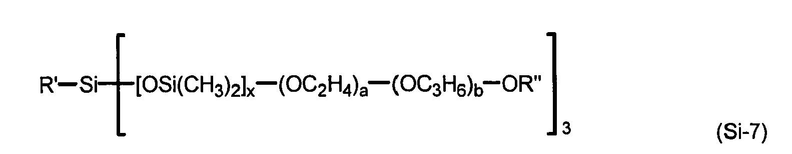 worin
- der Rest R steht für ein Wasserstoffatom, eine Alkylgruppe mit 1 bis 12 C-Atomen, eine Alkoxygruppe mit 1 bis 12 C-Atomen oder eine Hydroxylgruppe,
- die Reste R' und R" bedeuten Alkylgruppen mit 1 bis 12 C-Atomen,
- x steht für eine ganze Zahl von 1 bis 100, bevorzugt von 20 bis 30,
- y steht für eine ganze Zahl von 1 bis 20, bevorzugt von 2 bis 10 und
- a und b stehen für ganze Zahlen von 0 bis 50, bevorzugt von 10 bis 30.

Verbindungen die unter die oben genannten Formeln fallen, werden in den folgenden Patentanmeldungen, auf die explizit Bezug genommen wird, offenbart: US-A-4,122,029; US-A-4,265,878; US-A-4,421,769 und GB-A-2,066,659.

Besonders bevorzugte Dimethiconcopolyole als nichtionische Tenside im Sinne der Erfindung sind beispielsweise die kommerziell unter dem Handelsnamen SILWET (Union Carbide Corporation) und DOW CORNING (Dow) vertriebenen Produkte. Erfindungsgemäß besonders bevorzugte Dimethiconcopolyole sind Dow Corning 190 und Dow Corning 193 (Dow).

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethyl-carboxymethylglycinat. Ein bevorzugtes zwitterionisches Tensid ist das unter der INCI-Be-zeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobutter-säuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß können als kationische Tenside insbesondere solche vom Typ der quartären Ammoniumverbindungen, der Esterquats und der Amidoamine eingesetzt werden.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCl-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Marken Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Marke Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die zusätzliche Verwendung mindestens eines (gegebenenfalls ethoxylierten und/oder propoxylierten) (C₈ bis C₃₀)-Alkylamids des eines Aminoalkohols, insbesondere mindestens einer Verbindung der Formel (Va) und/oder mindestens einer Verbindung der Formel (Vb), bewirkt eine zusätzliche Verbesserung des erfindungsgemäßem Effektes, 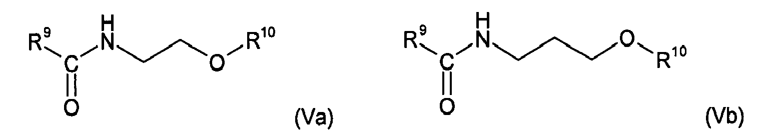 worin bedeuten
- R⁹: eine lineare oder verzweigte (C₈ bis C₃₀)-Alkylgruppe und
- R¹⁰: ein Wasserstoffatom, eine Gruppe -(CH₂CH₂O)ₙH mit n = 2 bis 10, eine Gruppe -(CH₂CH₂CH₂O)mH mit m = 2 bis 10, eine Gruppe -(CH₂CH₂O)ₙ(CH₂CH₂CH₂O)ₘH mit n = 2 bis 10 und m = 2 bis 10, eine Gruppe -(CH₂CH₂CH₂O)ₘ,(CH₂CH₂O)ₙH mit n = 2 bis 10 und m = 2 bis 10.

Daher ist es erfindungsgemäß bevorzugt, wenn die erfindungsgemäßen Mittel zusätzlich mindestens ein (gegebenenfalls ethoxylierten und/oder propoxylierten) (C₈ bis C₃₀)-Alkylamids des eines Aminoalkohols (insbesondere mindestens eine Verbindung der obigen Formel (Va) und/oder mindestens eine Verbindung der obigen Formel (Vb)), enthalten. Dabei ist es wiederum bevorzugt, wenn in dem erfindungsgemäßen Mittel mindestens ein anionisches Tensid mit mindestens einem (gegebenenfalls ethoxylierten und/oder propoxylierten) (C₈ bis C₃₀)-Alkylamid des eines Aminoalkohols kombiniert wird.

Verbindungen der Formel (Va) eignen sich dabei erfindungsgemäß bevorzugt, und zwar wiederum vorzugsweise solche, in denen R¹⁰ für ein Wasserstoffatom oder eine Gruppe - (CH₂CH₂O)ₙH mit n = 2 bis 10 steht, als besonders bevorzugter Vertreter dieser Verbindungen ist das Handelsprodukt Comperlan^{®} 100 zu nennen (INCI-Bezeichnung: Cocamide MEA; ein Cocosfettsäureamid des 2-Aminoethanols, Amidgehalt 92-99 Gew.-%, Estergehalt maximal 5 Gew.-%).

Desweiteren können die erfindungsgemäßen Mittel zusätzlich mindestens ein Proteinhydrolysat enthalten. Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Keratin DEC^{®} (Vincience), Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Gluadin^{®} (Cognis), Lexein^{®} (Inolex), Crolastin^{®} (Croda) oder Crotein^{®} (Croda) vertrieben.

Bevorzugt sind die Proteinhydrolysate in einer Menge von 0,05 bis 5 Gew.%, besonders bevorzugt von 0,5 bis 2,0 Gew.-%, jeweils bezogen auf das Gewicht des erfindungsgemäßen Mittels, enthalten.

Das gebrauchsfertige erfindungsgemäße Mittel sollte bevorzugt einen pH-Wert im Bereich von pH 5 bis pH 12, insbesondere von pH 7 bis pH 11, aufweisen.

Als pH-Stellmittel enthalten die erfindungsgemäßen Mittel bevorzugt zusätzlich Säuren und/oder Alkalisierungsmittel.

Als Säuren werden erfindungsgemäß bevorzugt Phosphorsäure oder Genußsäuren, wie beispielsweise Zitronensäure, Weinsäure oder Äpfelsäure, eingesetzt.

Die erfindungsgemäß verwendbaren Alkalisierungsmittel und werden bevorzugt ausgewählt aus der Gruppe, die gebildet wird, aus Ammoniak, basischen Aminosäuren, Alkalihydroxiden, Alkanolaminen, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium. Wiederum bevorzugt sind die Alkalisierungsmittel von Ammoniak verschieden.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Besonders bevorzugt wird das Alkalisierungsmittel ausgewählt aus mindestens einer Verbindung aus der Gruppe, die gebildet wird aus 2-Aminoethanol, 2-Amino-2-methylpropan-1-ol, 2-Amino-2-methyl-propan-1,3-diol, Kaliumhydroxid, L-Arginin, D-Arginin, DL-Arginin, N-Methylglucamin, Morpholin, Imidazol und Harnstoff.

Ferner können die erfindungsgemäßen Färbemittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/lsobornylacrylat-Copolymere, Methylvinylether/Malein-säureanhydrid-Copolymere und Acrylsäure/Ethylacrylat/N-tert.Butyl-acrylamid-Terpolymere,
- Strukturanten wie Maleinsäure und Milchsäure,
- haarkonditionierende Verbindungen wie Phospholipide, beispielsweise Sojalecithin, Ei-Lecitin und Kephaline,
- Parfümöle, Dimethylisosorbid und Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Antischuppenwirkstoffe wie Piroctone Olamine, Zink Omadine und Climbazol,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆ C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel; Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Dieerfindungsgemäßen Mittel können beispielsweise als Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind, formuliert sein.

Das erfindungsgemäße Mittel kann in einer Verpackungseinheit (Kit), welche mindestens ein getrennt konfektioniertes, erfindungsgemäßes Mittel des zweiten Erfindungsgegenstandes enthält, bereitgestellt werden. Zusätzlich kann das Kit mindestens eine getrennt konfektionierte Oxidationsmittelzusammensetzung enthalten, insbesondere dann, wenn ein (oxidatives) Färbemittel bereitgestellt werden soll. Darüber hinaus kann das Kit zusätzlich optional eine Gebrauchsanweisung, Applikationshilfen, Anmischgefäße oder Schutzhandschuhe enthalten.

Ein zweiter Gegenstand der vorliegenden Erfindung ist ein Verfahren zur Behandlung keratinhaltiger Fasern, bei dem ein Mittel des ersten Erfindungsgegenstandes auf die Fasern aufgetragen wird und nach einer Einwirkungszeit wieder abgespült wird.

Die Anwendungstemperaturen können in einem Bereich zwischen 15 und 40 °C liegen. Nach einer Einwirkungszeit von in der Regel 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Ein dritter Gegenstand der Erfindung ist die Verwendung eines Mittels des ersten Erfindungsgegenstandes zur Färbung von keratinhaltigen Fasern, insbesondere von menschlichem Haar und gleichzeitigem Schutz dieser Färbung vor dem Ausbleichen durch Licht, insbesondere durch UV-Licht.

Ein vierter Erfindungsgegenstand ist ein Verfahren zur Herstellung eines Mittels des ersten Erfindungsgegenstandes, worin in einem ersten Gefäß mindestens eine farbgebende Komponente in eine wässrige Phase eingearbeitet wird, in einem zweiten Gefäß mindestens ein organischer UV-Absorber in mindestens einen Fettstoff eingearbeitet wird, und die Inhalte des ersten und des zweiten Gefäßes gemischt werden.

### Beispiele

Alle Mengenangaben sind - wenn nicht anders gekennzeichnet - Gewichtsprozent. Folgende Handelsprodukte wurden als Rohstoffe verwendet:

| | |
|---|---|
| Texapon^{®} NSO UP | Laurylethersulfat, Natriumsalz (ca. 27,5% Aktivsubstanz; INCI-Bezeichnung: Sodium Laureth Sulfate) (Cognis) |
| Stenol^{®} 1618 | C₁₆₋₁₈-Fettalkohol (INCI-Bezeichnung: Cetearyl Alcohol) (Cognis) |
| Dehyquart^{®} A-CA | Trimethylhexadecylammoniumchlorid (ca. 24 -26 % Aktivsubstanz; INCI-Bezeichnung: Aqua (Water), Cetrimonium Chloride) (Cognis) |
| Comperlan^{®} 100 | Cocosfettsäuremonoethanolamid (95% Amidgehalt, 5% Ester der Cocosfettsäure mit Monoethanolamin, Feststoff, INCI-Bezeichung: Cocamide MEA) (Cognis) |
| Lorol^{®} tech. | C₁₂₋₁₈-Fettalkohol (INCI-Bezeichnung: Coconut alcohol) (Cognis) |
| Edenor^{®} PK 1805 | Ölsäure (INCI-Bezeichnung: Oleic Acid) (Cognis) |
| Turpinal^{®} SL | 1-Hydroxyethan-1,1-diphosphonsäure (ca. 58 - 61% Aktivsubstanzgehalt; INCI-Bezeichnung: Etidronic Acid, Aqua (Water)) (Solutia) |
| Gluadin^{®} W40 | Weizenproteinhydrolysat (mind. 40% Festkörper; INCI-Bezeichnung: Aqua (Water), Hydrolyzed Wheat Protein, Sodium Benzoate, Phenoxyethanol, Methylparaben, Propylparaben) (Cognis) |
| Tinogard^{®} TL | 2-(2H-Benzotriazol-2-yl)-6-dodecyl-4-methylphenol (Gemisch aus Derivaten mit linearer und verzweigter Dodecylgruppe; gelbe viskose Flässigkeit; INCl-Bezeichnung: Benzotriazolyl Dodecyl p-Cresol) (Ciba) |
| Emulgade^{®} F | Mischung aus Cetylstearylalkohol, Rizinusöl mit ca. 40-EO-Einheiten und dem Natriumsalz von Cetylstearylsulfat (INCI-Bezeichnung: Cetearyl Alcohol, PEG-40 Castor Oil, Sodium Cetearyl Sulfate) (Cognis) |

### 1. Herstellung der Färbecremes

Tinogard TL, Stenol 1618 und Lorol techn. wurden in Gefäß A bei einer Temperatur von 58°C innig gemischt. Zu dieser Mischung wurde Dehyquart A CA gegeben. In einem separaten Gefäß B wurden die Farbstoffe bzw. Farbstoffvorprodukte bei Raumtemperatur in Wasser gelöst und die übrigen Inhaltsstoffe der Reihenfolge nach unter Rühren zugefügt. Abschließend wurde die Mischung aus Gefäß A zu der Mischung des Gefäßes B gegeben.

Die Zusammensetzung der jeweiligen Färbecreme F1 bis F5 ist der nachfolgenden Tabelle 1 zu entnehmen.

**Tabelle 1: Färbecremes F1 bis F5**

| | F1 | F2 | F3 | F4 | F5 |
|---|---|---|---|---|---|
| Texapon NSO UP | 25,00 | 25,00 | 25,00 | 25,00 | 25,00 |
| Stenol1618 | 9,00 | 9,00 | 9,00 | 9,00 | 9,00 |
| Dehyquart A CA | 3,00 | 3,00 | 3,00 | 3,00 | 3,00 |
| Comperlan 100 | 2,50 | 2,50 | 2,50 | 2,50 | 2,50 |
| Lorol techn. | 2,00 | 2,00 | 2,00 | 2,00 | 2,00 |
| Isostearinsäure | 1,00 | 1,00 | 1,00 | 1,00 | 1,00 |
| Edenor PK 1805 | 1,00 | 1,00 | 1,00 | 1,00 | 1,00 |
| KOH (50%ige Lösung in Wasser) | 1,00 | 1,00 | 1,00 | 1,00 | 1,00 |
| Tinogard TL | 0,50 | 0,50 | 0,50 | 0,50 | 0,50 |
| 2-Aminoethanol | 4,00 | 4,00 | 4,00 | 4,00 | 4,00 |
| Schwefelsäure (20%ig in Wasser) | 0,01 | 0,01 | 0,01 | 0,01 | 0,01 |
| Ascorbinsäure | 0,10 | 0,10 | 0,10 | 0,10 | 0,10 |
| Natriumsulfit | 0,20 | 0,20 | 0,20 | 0,20 | 0,20 |
| Turpinal SL | 0,20 | 0,20 | 0,20 | 0,20 | 0,20 |
| Natronwasserglas 40/42 | 0,50 | 0,50 | 0,50 | 0,50 | 0,50 |
| Gluadin W40 | 0,60 | 0,60 | 0,60 | 0,60 | 0,60 |
| p-ToluylendiaminH₂SO₄ | 0,50 | 0,91 | 1,12 | 1,67 | 1,62 |
| 2,4,5,6-Tetraaminopyrimidin H₂SO₄ | - | 0,42 | - | - | - |
| 4-Amino-3-methylphenol | - | 0,03 | 0,01 | - | - |
| 4,5-Diamino-1-(2-hydroxyethyl)pyrazolsulfat | - | 1,14 | - | - | - |
| Resorcin | - | 0,08 | 0,56 | 0,22 | 0,41 |
| m-Aminophenol | 0,02 | 0,10 | 0,08 | 0,35 | 0,41 |
| 1-Naphthol | 0,01 | - | - | - | - |
| 2,7-Dihydroxynaphthalin | - | 0,12 | - | - | - |
| 5-Amino-2-methylphenol | 0,06 | 0,60 | 0,04 | - | 0,08 |
| 2-Methylresorcin | 0,09 | 0,26 | - | - | 0,18 |
| 1,3-Bis(2,4-diaminophenoxy)propan | - | - | - | 0,43 | - |
| 2-Amino-4-(2-hydroxyethyl)aminoanisol | - | - | - | - | 0,05 |
| 3-Amino-2-methylamino-6-methoxy-pyridin | - | - | - | 0,50 | - |
| 2-Amino-3-hydroxypyridin | 0,08 | - | - | - | - |
| 2-Amino-6-chlor-4-nitrophenol | 0,12 | - | - | - | 0,05 |
| Wasser | ad 100 | ad 100 | ad 100 | ad 100 | ad 100 |

### 2. Ausfärbungen

Zur Herstellung des anwendungsbereiten Färbemittels wurden 50 g der Färbecreme unter Rühren mit 20 g des Entwicklers gemäß Tabelle 2 gemischt.

**Tabelle 2: Entwickler**

| | |
|---|---|
| Phosphorsäure (85%ig in Wasser) | 0,04 |
| Dinatriumpyrophosphat | 0,30 |
| Dinatrium EDTA | 0,15 |
| Emulgade F | 2,10 |
| Natriumbenzoat | 0,04 |
| Wasserstoffperoxid | 3,00 |
| Aqua | ad 100 |

Bei Applikation auf eine Haartresse (Kerling Euronaturhaar 6/0) und einer Einwirkungszeit von 30 Minuten wurde nach dem Spülen und Trocknen ein hervorragendes Färbeergebnis bei guter Lichtechtheit der Färbung erhalten.

## Patentansprüche

1. Mittel zur Färbung keratinhaltiger Fasern, insbesondere menschlicher Haare, enthaltend
(i) eine wässrige Phase,
(ii) mindestens einen Fettstoff als Fettphase,
(iii) mindestens einen organischen UV-Absorber und
(iv) mindestens eine farbverändernde Komponente,
mit der Maßgabe, dass sich mindestens ein organischer UV-Absorber in mindestens einem Fettstoff der Fettphase befindet und die farbverändernden Komponenten sich in der wässrigen Phase befinden.

2. Mittel nach Anspruch 1, **dadurch gekennzeichnet, daß** der Fettstoff ausgewählt wird, aus Silikonen, Fettsäuren, Fettalkoholen, natürlichen oder synthetischen Wachsen und natürlichen oder synthetischen kosmetischen Ölkomponenten, sowie Mischungen von Verbindungen aus diesen Stoffklassen.

3. Mittel nach einem der Ansprüche 1 oder 2, **dadurch gekennzeichnet, daß** die Fettsäuren ausgewählt werden aus mindestens einer linearen und/oder verzweigten, gesättigten und/oder ungesättigten Fettsäure mit 6 bis 30 Kohlenstoffatomen.

4. Mittel nach einem der Ansprüche 1 bis 3, **dadurch gekennzeichnet, daß** die Fettalkohole ausgewählt werden aus gesättigten, ein- oder mehrfach ungesättigten, verzweigten oder unverzweigten Fettalkoholen mit 6 bis 30 Kohlenstoffatomen, bevorzugt mit 10 bis 22 Kohlenstoffatomen und ganz besonders bevorzugt mit 12 bis 22 Kohlenstoffatomen.

5. Mittel nach einem der Ansprüche 1 bis 4, **dadurch gekennzeichnet, daß** die natürlichen oder synthetischen Wachse ausgewählt werden, aus mindestens einem Wachs der Gruppe, die gebildet wird, festen Paraffinen oder Isoparaffinen, Carnaubawachs, Bienenwachs, Candelillawachs, Ozokerit, Ceresin, Walrat, Sonnenblumenwachs, Fruchtwachs wie beispielsweise Apfelwachs oder Citruswachs, Microwachse aus Polyethylen oder Polypropylen.

6. Mittel nach einem der Ansprüche 1 bis 5, **dadurch gekennzeichnet, daß** die farbverändernde Komponente ausgewählt wird,
(1) aus mindestens einem Oxidationsfarbstoffvorprodukt vom Typ der Entwicklerkomponenten und gegebenenfalls zusätzlich mindestens einer Kupplerkomponente
und/oder
(2) aus Oxofarbstoffvorprodukten
und/oder
(3) aus mindestens einem direktziehenden Farbstoff
und/oder
(4) aus mindestens einer Vorstufe naturanaloger Farbstoffe.

7. Mittel nach einem der Ansprüche 1 bis 6, **dadurch gekennzeichnet, daß** die organischen UV-Absorber Die in einer Menge von 0,05 bis 10,0 Gew.-% insbesondere von 0,1 bis 5,0 Gew.-%, jeweils bezogen auf das Gewicht des Färbemittels, enthalten sind.

8. Mittel nach einem der Ansprüche 1 bis 7, **dadurch gekennzeichnet, daß** als organischer UV-Absorber mindestens eine Verbindung der Formel (1) enthalten ist, 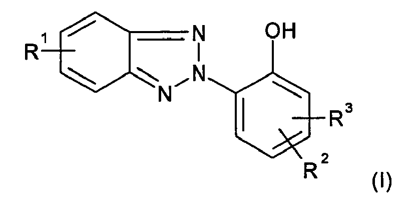 worin
R¹ steht für ein Wasserstoffatom, ein Chloratom, ein Bromatom, eine Hydroxygruppe, eine (C₁ bis C₄)-Alkoxygruppe oder eine (C₁ bis C₄)-Alkylgruppe,
R² steht für eine lineare oder verzweigte (C₁ bis C₃₀)-Alkylgruppe, eine Arylgruppe, eine Aryl-(C₁ bis C₄)-alkylgruppe, eine (C₅ bis C₇)-Cycloalkylgruppe, eine (C₁ bis C₄)-Alkoxycarbonylgruppe, eine Sulfonylgruppe, eine Sulfonatgruppe oder eine Sulfonsäureester-Gruppe,
R³ steht für eine (C₁ bis C₃₀)-Alkylgruppe, eine lineare oder verzweigte (C₁ bis C₃₀)-Acyloxygruppe eine (C₈ bis C₂₂)-Alkenylgruppe oder eine Hydroxybenzylgruppe der Formel (II), 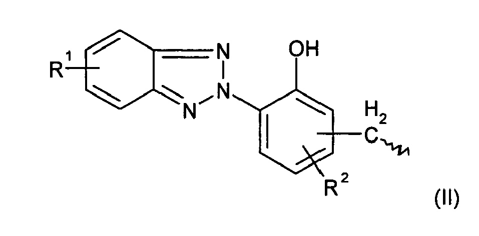
worin R¹ und R² wie oben definiert sind.

9. Mittel nach Anspruch 8, **dadurch gekennzeichnet, daß** die organischen UV-Absorber der Formel (I) ausgewählt werden aus mindestens einer Verbindung der Gruppe, die gebildet wird, aus 2-(2H-Benzotriazol-2-yl)-4-methyl-5-dodecylphenol, 2-(2H-Benzotriazol-2-yl)-4-methyl-6-dodecylphenol, 2-(2H-Benzotriazol-2-yl)-4-tert-butylphenol, 2-(5-Chloro-2H-benzotriazol-2-yl)-6-tert-butyl-4-methylphenol, 2,2'-Methylen-bis(6-(2H-benzotriazol-2-yl)-4-(1,1,3,3-tetramethylbutyl)phenol), 2-(2H-Benzotriazol-2-yl)-4-methylphenol, 2-(5-Chloro-2H-benzotriazol-2-yl)-4,6-di(tert-butyl)phenol, 2-(2H-Benzotriazol-2-yl)-4-methyl-6-C₁₂H₂₅-phenol, Natrium 3-(2H-Benzotriazol-2-yl)-4-hydroxy-5-(sec-butyl)benzolsulfonat, 2-(2H-Benzotriazol-2-yl)-4-tert-butyl-6-sec-butylphenol, 2-(2H-Benzotriazol-2-yl)-4-(1,1,3,3-tetramethylbutyl)-phenolsulfonsäureester, 2-(2H-Benzotriazol-2-yl)-4-(1,1,3,3-tetramethylbutyl)phenol, 2-(2H-Benzotriazol-2-yl)-4,6 bis(1,1,3,3-tetramethylbutyl)phenol, 2-(2H-Benzotriazol-2-yl)-4,6 bis(tert-pentyl)phenol und 2-(2H-Benzotriazol-2-yl)-4,6-bis(2-phenylprop-2-yl)phenol.

10. Mittel nach einem der Ansprüche 1 bis 9, **dadurch gekennzeichnet, daß** als organischer UV-Absorber mindestens ein Derivat des Benzophenons gemäß Formel (III) enthalten ist, 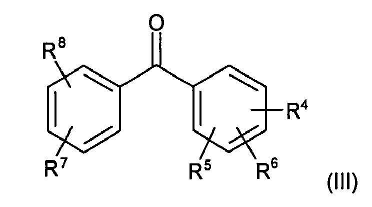 worin
R⁴, R⁵, R⁶, R⁷ und R⁸ unabhängig voneinander stehen für ein Wasserstoffatom, ein Halogenatom (insbesondere ein Chloratom), eine Hydroxygruppe, eine (C₁ bis C₁₀)-Alkyloxygruppe, eine Sulfonsäuregruppe oder eine Sulfonatgruppe, mit der Maßgabe, dass mindestens ein Rest aus R⁴, R⁵, R⁶, R⁷ und R⁸ von einem Wasserstoffatom verschieden ist.

11. Mittel nach Anspruch 10, **dadurch gekennzeichnet, daß** die organischen UV-Absorber der Formel (III) ausgewählt werden aus mindestens einer Verbindung aus der Gruppe, die gebildet wird, aus 2,4-Dihydroxybenzophenone (Benzophenone 1), 2,2',4,4'-Tetrahydroxybenzophenon (Benzophenone 2), 2-Hydroxy-4-(methoxy)benzophenon (Benzophenone 3), 2-Hydroxy-4-methoxybenzophenon-5-sulfonsäure (Benzophenone 4) bzw. ein Salz davon, 2,2'-Dihydroxy-4,4'-dimethoxybenzophenon (Benzophenone 6), 5-Chlor-2-hydroxybenzophenon (Benzophenone 7), 2,2'-Dihydroxy-4-methoxybenzophenon, 2,2'-Dihydroxy-4-octyloxybenzophenon, 2-Hydroxy-4-(octyloxy)benzophenon (Benzophenone 12).

12. Mittel nach einem der Ansprüche 1 bis 11, **dadurch gekennzeichnet, daß** es zusätzlich mindestens ein Tensid enthält.

13. Mittel nach einem der Ansprüche 1 bis 12, **dadurch gekennzeichnet, daß** es zusätzlich mindestens ein (gegebenenfalls ethoxyliertes und/oder propoxyliertes) (C₈ bis C₃₀)-Alkylamid eines Aminoalkohols enthält.

14. Mittel nach Anspruch 13, **dadurch gekennzeichnet, daß** das (gegebenenfalls ethoxylierte und/oder propoxylierte) (C₈ bis C₃₀)-Alkylamid eines Aminoalkohols ausgewählt wird aus mindestens einer Verbindung der Formel (Va) und/oder mindestens einer Verbindung der Formel (Vb), 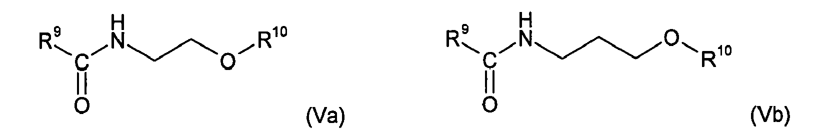 worin bedeuten
R⁹ eine lineare oder verzweigte (C₈ bis C₃₀)-Alkylgruppe und
R¹⁰ ein Wasserstoffatom, eine Gruppe -(CH₂CH₂O)ₙH mit n = 2 bis 10, eine Gruppe -(CH₂CH₂CH₂O)ₘH mit m = 2 bis 10, eine Gruppe -(CH₂CH₂O)ₙ(CH₂CH₂CH₂O)ₘH mit n = 2 bis 10 und m = 2 bis 10, eine Gruppe -(CH₂CH₂CH₂O)ₘ(CH₂CH₂O)ₙH mit n = 2 bis 10undm=2bis10.

15. Verfahren zur Behandlung keratinhaltiger Fasern, bei dem ein Mittel nach einem der Ansprüche 1 bis 14 auf die Fasern aufgetragen wird und nach einer Einwirkungszeit wieder abgespült wird.

16. Verwendung eines Mittels nach einem der Ansprüche 1 bis 14 zur Färbung von keratinhaltigen Fasern, insbesondere von menschlichem Haar und gleichzeitigem Schutz dieser Färbung vor dem Ausbleichen durch Licht, insbesondere durch UV-Licht.

17. Verfahren zur Herstellung eines Mittels nach einem der Ansprüche 1 bis 11, **dadurch gekennzeichnet, daß** in einem ersten Gefäß mindestens eine farbgebende Komponente in eine wässrige Phase eingearbeitet wird, in einem zweiten Gefäß mindestens ein organischer UV-Absorber in mindestens einen Fettstoff eingearbeitet wird, und die Inhalte des ersten und des zweiten Gefäßes gemischt werden.
